# FASCICULE DE BREVET EUROPEEN

(11) **EP 2 470 158 B1**
(45) Date de publication et mention de la délivrance du brevet: **05.10.2016**
(21) Numéro de dépôt: 10762993.3
(22) Date de dépôt: 18.08.2010
(51) Int. Cl.: A61K 8/35, A61K 8/58, A61Q 17/04, A61K 8/41, A61K 8/49

(54) **COMPOSITION CONTENANT AU MOINS UN FILTRE DU TYPE 2- HYDROXYBENZOPHENONE LIPOPHILE ET UNE S-TRIAZINE SILICIEE SUBSTITUEE PAR AU MOINS DEUX GROUPES ALKYLAMINOBENZOATES**
ZUSAMMENSETZUNG ENTHALTEND EINEN LIPOPHILEN 2-HYDROXYBENZOPHENON-FILTER UND EIN SILIZIERTES S-TRIAZIN DAS MIT MINDESTENS ZWEI ALKYLAMINOBENZOAT-GRUPPEN SUBSTITUIERT IST
COMPOSITION CONTAINING AT LEAST ONE LIPOPHILIC 2-HYDROXYBENZOPHENONE FILTER AND A SILICON S-TRIAZINE SUBSTITUTED BY AT LEAST TWO ALKYLAMINOBENZOATE GROUPS

(30) Priorité: 28.08.2009 FR 0955882; 28.08.2009 FR 0955883; 04.09.2009 US 272261 P; 04.09.2009 US 272260 P
(43) Date de publication de la demande: 04.07.2012
(73) Titulaire: L'Oréal, 75008 Paris (FR)
(72) Inventeur: RICHARD, Hervé, F-93320 Gagny (FR); L'ALLORET, Florence, F-75014 Paris (FR); CANDAU, Didier, F-91570 Bievres (FR); FIANDINO Cécile, F-75013 Paris (FR)
(74) Mandataire: Nony
(86) Numéro de dépôt international: PCT/FR2010/051730
(87) Numéro de publication internationale: WO 2011/023886

(56) Documents cités:
- EP-A2- 0 843 996
- EP-A2- 0 864 313
- EP-A2- 1 046 391
- WO-A2-2007/071584
- FR-A1- 2 886 143

## Description

La présente invention concerne une composition comprenant dans un support cosmétiquement acceptable au moins un système filtrant UV, caractérisée par le fait qu'elle comprend :
(i) au moins un filtre de type 2-hydroxybenzophénone lipophile en une teneur variant de 0,1 à 10 % en poids par rapport au poids total de la composition ;
(ii) au moins une s-triazine siliciée de formule (III) substituée par au moins deux groupes alkylaminobenzoates.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

Dans le but d'assurer une protection de la peau et des matières kératiniques contre le rayonnement UV, on utilise généralement des compositions antisolaires comprenant des filtres organiques, actifs dans l'UV-A et actifs dans l'UV-B.

On sait les dérivés de benzophénone lipophiles sont connus pour être des filtres photostables absorbant les radiations UVA et certains d'entre eux comme les 2-hydroxybenzophénone aminosubstitués comme le le 2-(4-diéthylamino-2-hydroxybenzoyl)-benzoate de n-hexyle, vendu sous la dénomination d'Uvinul A + par la société BASF ont une efficacité élevée.

Des filtres UV-B particulièrement intéressants et largement utilisés à ce jour sont les filtres du type 1,3,5-triazine non siliciés lipophiles. Ils sont décrits dans les demandes de brevets EP-A-0517104, EP-A-0570838, EP-A-796851 et EP-A-0775698. On connaît en particulier le dérivé 2,4,6-tris[p-(2'-éthylhexyl-1'-oxy-carbonyl)anilino]-1,3,5-triazine vendu notamment sous la dénomination commerciale de « Uvinul T150 » par la société BASF. Ces filtres lipophiles actifs dans l'UVB présentent le désavantage d'être solide à température ambiante. Leur utilisation dans des compositions solaires induit des contraintes de formulation, avec la nécessité d'identifier des solvants permettant de les solubiliser correctement.

D'autres filtres UV-B intéressants et utilisés dans les compositions solaires à ce jour sont les β,β'-diphénylacrylate d'alkyle ou α-cyano-β,β'-diphénylacrylate d'alkyle; Citons le α-cyano-β,β' diphénylacrylate de 2-éthylhexyle, encore appelé octocrylène. Il est disponible commercialement et vendu notamment sous la dénomination de "UVINUL N 539" par la Société BASF. Ces filtres présentent l'avantage d'être facilement formulables mais leurs propriétés filtrantes sont relativement faibles, ce qui se traduit par des concentrations d'utilisation élevées pour obtenir un niveau de filtration UV intéressant.

Les dérivés de cinnamate tels que le 4-méthoxycinnamate d'éthyl-2-hexyle ou le 4-méthoxycinnamate d'isoamyle sont de bons solvants de filtres UV difficilement solubles dans les huiles et présentent de bonnes propriétés de photoprotection dans l'UVB. Ces dérivés de cinnamate ont l'inconvénient de présenter une photostabilité insuffisante et de perturber la photostabilité des systèmes filtrants complets dans lesquels ils sont introduits.

On connaît dans les demandes EP0841341 et la demande EP1891079 des s-trazines siliciées substitués par au moins deux groupes alkylaminobenzoates ou alkylaminobenzamides pour leurs propriétés absorbantes dans l'UV-B. La demanderesse au cours de ses recherches a constaté que certains de ces composés triazines siliciées substituées par au moins deux groupes alkylaminobenzoates notamment celles dont la chaîne alkyle comporte plus de 10 atomes de carbone ne permettaient pas d'obtenir une efficacité suffisante en association avec des filtres UVA.

Il apparaît ainsi nécessaire de disposer d'un système filtrant de façon large le rayonnement UVA et UVB, facile à mettre en oeuvre dans des compositions solaires sans les inconvénients évoqués ci-dessus à savoir obtenir un système filtrant les rayons UVA et UVB qui soit photostable, d'indice de protection élevé et ayant une bonne solubilité dans les formulations solaires.

Or, la Demanderesse vient maintenant de découvrir, de façon surprenante, qu'en associant au moins un filtre de type 2-hydroxybenzophénone lipophile avec une s-triazine siliciée substituée par au moins deux groupes alkylaminobenzoates de formule (III) dont on donnera la définition plus loin, il était possible d'obtenir un système filtrant de façon large le rayonnement UVA et UVB, facile à mettre en oeuvre dans des compositions solaires sans les inconvénients évoqués ci-dessus.

Cette découverte est à la base de la présente invention.

Ainsi, conformément à l'un des objets de la présente invention, il est maintenant proposé une composition comprenant dans un support cosmétiquement acceptable au moins un système filtrant UV, caractérisée par le fait qu'elle comprend :
(ii) au moins un filtre de type hydroxybenzophénone lipophile en une teneur variant de 0,1 à 10 % en poids par rapport au poids total de la composition ;
(iii) au moins une s-triazine siliciée de formule (III) substituée par au moins deux groupes alkylaminobenzoates.

Plus particulièrement la présente invention a pour objet une composition comprenant dans un support cosmétiquement acceptable au moins un système filtrant UV, caractérisée par le fait qu'elle comprend :
(i) au moins un filtre de type hydroxybenzophénone lipophile répondant à la formule (I) suivante : 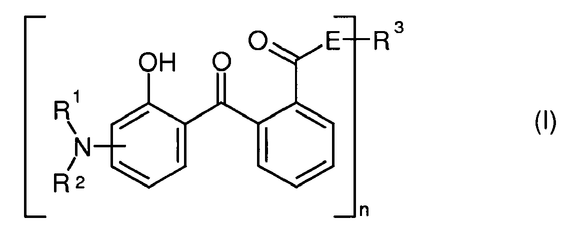 dans laquelle :
   R¹ et R², identiques ou différents, désignent un radical alkyle en C₁-C₂₀, un alcényle en C2-C20, un cycloalkyle en C₃-C₁₀, un cycloalcényle en C₃-C₁₀ ou forment avec l'atome d'azote avec lequel ils sont liés un cycle à 5 ou 6 chaînons ;
   n est un nombre allant de 1 à 4 avec
      quand n = 1, R² désigne un radical alkyle ou alcényle en C₁-C₂₀, un hydroxyalkyle en C₁-c₅, un cyclohexyle en C₆-C₁₂, un phényle pouvant être substitué par O. N ou S, aminocarbonyle ou alkylcarbonyle en C₁-C₅ ;
      quand n = 2, R³ désigne un diradical alkyle, un diradical cycloalkyle, un diradical alcényle ou un diradical aryle ou R³ avec E forment un diradical de formule (II) : 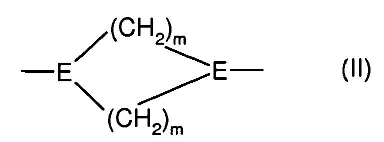 avec m un nombre allant de 1 à 3 ;
      quand n = 3, R³ est un triradical alkyle ;
      quand n = 4 , R³ est un tetraradical alkyle ;
   E est -O-, ou -N(R⁴)- ;
   R4 est hydrogène, un radical alkyle en C₁-C₅ ou hydroxyalkyle en C₁-C₅ ; le ou les filtres lipophiles 2-hydroxybenzophénone étant présents à des teneurs variant de 0.1 à 10 % en poids par rapport au poids total de la composition ,
(ii) au moins une s-triazine siliciée de formule générale (III) suivante ou l'une de ses formes tautomères : 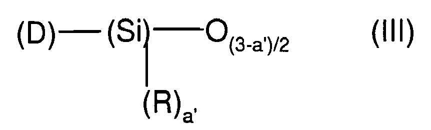 dans laquelle
   - R, identiques ou différents représentent un radical alkyle en C₁-C₂, un radical phényle, un radical alkoxy en C₁-C₂, un radical hydroxyl ou le groupe triméthylsilyloxy ;
   - a' = 1 à 3 ;
   - le groupe (D) désigne un composé s-triazine de formule (IV) suivante : 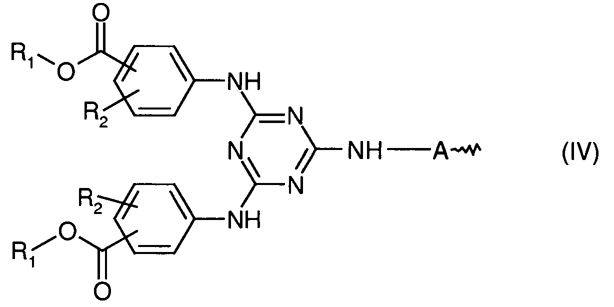 où
   - R₁, identiques ou différents, représentent un radical alkyle en C₁-C₁₀, linéaire ou ramifié et éventuellement insaturé et pouvant contenir un groupe cycloalkyle en C₅-C₆,
   - le groupement (C=O)OR₁ pouvant être en position ortho, méta ou para du groupement amino,
   - R₂, identiques ou différents, représentent hydrogène, un radical hydroxyl, un radical alkyle en C₁-C₄, linéaire ou ramifié, un radical méthoxy,
   - A est un radical divalent choisi parmi méthylène ou un groupe répondant à l'une des formules (V), (VI) , (VII) ou (VIII) suivantes : 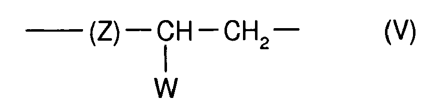 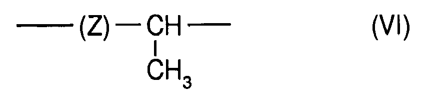 -(Z)-CH=CH- (VII) 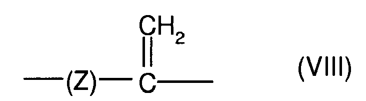 dans lesquelles :
   - Z est un diradical alkylène en C₁-C₃,
   - W représente un atome d'hydrogène, un radical hydroxyle ou un radical alkyle en C₁-C_{3 ;}
      en plus des unités de formule -A-(Si)(R)_{a'}(O)_{(3-a')/2}, l'organosiloxane peut comporter des unités de formule : (R)_{b}-(Si)(O)_{(4-b)/2} dans lesquelles :
      R a la même signification que dans la formule (I), b = 1, 2 ou 3, ou au moins une s-triazine siliciée répondant à l'une des formules (IIIa) ou (IIIb) suivantes : 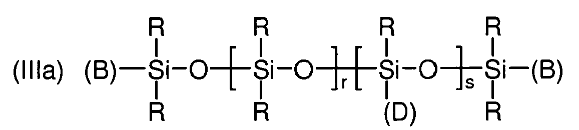 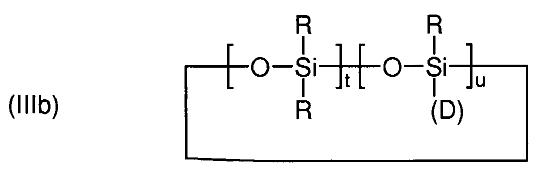 dans lesquelles :
   - (D) répond à la formule (IV) telle que définie ci-dessus,
   - R a la même définition que dans la formule (III) telle que définie ci-dessus,
   - (B), identiques ou différents sont choisis parmi les radicaux R et le radical (D),
   - r est un nombre entier compris entre 0 et 20 inclusivement,
   - s est un nombre entier allant de 0 à 5 et si s = 0, au moins l'un des deux symboles (B) désigne (D),
   - u est un nombre entier allant de 1 à 5,
   - t est un nombre entier allant de 0 à 5, étant entendu que t + u est égal ou supérieur à 3 ainsi que leurs formes tautomères.

D'autres caractéristiques, aspects et avantages de l'invention apparaîtront à la lecture de la description détaillée qui va suivre.

Par "cosmétiquement acceptable", on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Par "composé lipophile" on entend tout composé cosmétique ou dermatologique susceptible d'être complètement dissous à l'état moléculaire dans une phase grasse liquide ou bien d'être solubilisé sous forme colloïdale (par exemple sous forme micellaire) dans une phase grasse liquide.

La demanderesse a également découvert de manière surprenante que l'utilisation d'un filtre actif dans l'UV-B de type s-triazine siliciée substituée par deux groupements aminobenzoates de formule (III) que l'on définira plus loin en détail en association avec un filtre UVA de type 2-hydroxybenzophénone lipophile en présence de dérivé de dibenzoylméthane permettait d'obtenir une bonne photostabilisation des dérivés du dibenzoylméthane vis-à-vis du rayonnement UV, associée à une bonne efficacité de protection dans l'UVB pour pouvoir assurer une protection complète et efficace de la peau face aux agressions liées à l'exposition au rayonnement UV constitue, à ce jour, un problème qui n'a pas encore été résolu de manière complètement satisfaisante

En effet, les dérivés du dibenzoylméthane, et notamment le 4-ter-butyl-4'-méthoxydibenzoyl méthane sont connus à titre de filtres actifs dans les UV-A et sont notamment décrits dans les demandes de brevets français FR-A-2326405 et FR-A-2440933, ainsi que dans la demande de brevet européen EP-A-0114607. Malheureusement, il se trouve que les dérivés du dibenzoylméthane sont des produits relativement sensibles au rayonnement ultraviolet (surtout UV-A), c'est-à-dire qu'ils se dégradent plus ou moins rapidement sous l'action de ce dernier. Ainsi, ce manque substantiel de stabilité photochimique face au rayonnement ultraviolet auquel ils sont par nature destinés à être soumis, ne permet pas de garantir une protection constante durant une exposition solaire prolongée.

Plusieurs moyens de photostabilisation des dérivés de dibenzoylméthane sont connus à ce jour. Ainsi, il est connu dans la demande de brevet EP0514491, que les dérivés alkyl β, β '-diphenylacrylate ou α- cyano β, β '-diphenylacrylate améliore de manière substantielle la photostabilité des dérivés de dibenzoylméthane. Malheureusement si ces filtres possèdent une bonne efficacité vis-à-vis de l'amélioration de la photostabilité des dérivés de dibenzoylméthane, ils n'en présentent pas moins un pouvoir absorbant des UV-B médiocre.

Il est également connu dans la demande de brevet EP1280505, que le Bis-Ethylhexyloxyphenol Methoxyphenyl Triazine seul, améliore la photostabilité des dérivés de dibenzoylméthane. Malheureusement si ce filtre possède un fort pouvoir absorbant des UV-B il présente néanmoins une efficacité limitée vis-à-vis de l'amélioration de la photostabilité des dérivés de dibenzoylméthane ainsi une solubilité limitée dans les huiles.

On a proposé également dans les demandes EP0843996 et EP0864313 d'améliorer la photostabilité d'un dérivé de dibenzoylméthane par une 2-hydroxybenzophénone lipophile comme la 2-Hydroxy-4-Methoxybenzophenone ou benzophenone-3. Cette association ne permet pas d'obtenir une photostabilité du dibenzoylméthane pleinement suffisante ni d'obtenir une couverture large du spectre UV.

On connaît également dans les demandes de brevet EP1323411 et US66899461 décrivant des compositions solaires contenant un dérivé de 2-hydroxybenzophénone aminosubstitué associé à un dérivé de dibenzoylméthane dans un rapport en poids du dérivé de 2-hydroxybenzophénone aminosubstitué sur le dérivé de dibenzoylméthane supérieur à 1. Malheureusement si cette association possède une photostabilité améliorée elle se trouve fortement limitée au ratio des deux filtres et ne permet pas d'obtenir une couverture large du spectre UV

Il a été également proposé dans la demande EP1891079 d'améliorer la photostabilité des dérivés de dibenzoylméthane par des s-trazines siliciées substitués par au moins deux groupes alkylaminobenzoates ou alkylaminobenzamides. La demanderesse au cours de ses recherches a constaté que certains de ces composés triazines siliciées notamment ceux ayant des groupes alkylaminobenzoates dont la chaîne alkyle comporte plus de 10 atomes de carbone ne permettaient pas d'obtenir une efficacité suffisante.

Or, la Demanderesse vient maintenant de découvrir, de façon surprenante, que l'utilisation d'un filtre actif dans l'UV-B de type s-triazine siliciée substituée par deux groupements aminobenzoates de formule (III) que l'on définira plus loin en détail en association avec un filtre UVA de type 2-hydroxybenzophénone lipophile en présence de dérivé de dibenzoylméthane permettait de résoudre les problèmes techniques évoqués précédemment.

Ainsi, conformément à l'un des objets de la présente invention, il est maintenant proposé une composition comprenant dans un support cosmétiquement acceptable au moins un système filtrant UV, caractérisée par le fait qu'elle comprend :
(i) au moins un dérivé du dibenzoylméthane ;
(ii) au moins un filtre UV de type 2-hydroxybenzophénone lipophile en une teneur variant de 0,1 à 10 % en poids par rapport au poids total de la composition ; et
(iii) au moins une s-triazine siliciée substituée par au moins deux groupes alkylaminobenzoates de formule (III).

Elle concerne également un procédé de photostabilisation vis-à-vis du rayonnement d'au moins un filtre du type dérivé du dibenzoylméthane par une composition comprenant au moins au moins un filtre de type 2-hydroxybenzophénone lipophile en une teneur variant de 0,1 à 10 % en poids par rapport au poids de la composition et d'au moins une s-triazine siliciée substituée par au moins deux groupements aminobenzoates ou aminobenzamides de formule (III) définie ci-dessous.

Par « quantité efficace », on entend une quantité suffisante pour obtenir une amélioration notable et significative de la photostabilité du ou des dérivés du dibenzoylméthane dans la composition cosmétique. Cette quantité minimale en 2- hydroxy-benzophénone lipophile et s-triazine siliciée de formule (III), qui peut varier selon la nature du support retenu pour la composition, peut être déterminée sans aucune difficulté au moyen d'un test classique de mesure de photostabilité, tel que celui donné dans les exemples ci-après.

### FILTRES 2-HYDROXYBENZOPHENONE

Parmi les filtres du type 2-hydroxybenzophénone, on peut citer
- 2,4-Dihydroxybenzophenone avec comme nom INCI : Benzophenone-1 vendu sous le nom commercial « UVINUL 400 » par BASF,
- 2,2',4,4'-Tetrahydroxybenzophenone avec comme nom INCI : Benzophenone-2 vendu sous le nom commercial « UVINUL D50 » par BASF
- 2-Hydroxy-4-Methoxybenzophenone avec comme nom INCI : Benzophenone-3 ou Oxybenzone, vendu sous le nom commercial « UVINUL M40 » par BASF,
- 2,2'-Dihydroxy-4,4'-Dimethoxybenzophenone Benzophenone-6 vendu sous le nom commercial « Helisorb 11 » par Norquay
- 2,2'-Dihydroxy-4-Methoxybenzophenone avec comme nom INCI: Benzophenone-8 vendu sous le nom commercial « Spectra-Sorb UV-24 » par American Cyanamid
- 2-Hydroxy-4-Methoxy-4'-Methylbenzophenone avec comme nom INCI: Benzophenone-10,
- Hydroxy-4-(Octyloxy)Benzophenone avec comme nom INCI : Benzophenone-12.

Selon une forme particulièrement préférée de l'invention, on utilisera plus particulièrement les composés 2-hydroxybenzophénone amino- substitués de formule (I) suivante : 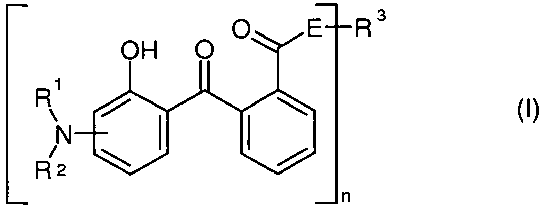 dans laquelle :
R¹ et R², identiques ou différents, désignent un radical alkyle en C₁-C₂₀ , un alcényle en C₂-C₂₀, un cycloalkyle en C₃-C₁₀ , un cycloalcényle en C₃-C₁₀ ou forment avec l'atome d'azote avec lequel ils sont liés un cycle à 5 ou 6 chaînons ;
n est un nombre allant de 1 à 4 ;
   quand n = 1, R³ désigne un radical alkyle ou alcényle en C₁-C₂₀, un hydroxyalkyle en C₁-C₅, un cyclohexyle en C₆-C₁₂, un phényle pouvant être substitué par O, N ou S, aminocarbonyle ou alkylcarbonyle en C₁-C₅ ;
   quand n = 2, R³ désigne un diradical alkyle, un diradical cycloalkyle, un diradical alcényle ou un diradical aryle ou R³ avec E forment un diradical de formule (II) : 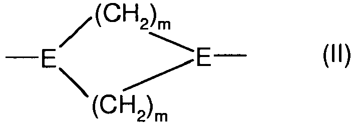 avec m un nombre allant de 1 à 3 ;
   quand n = 3, R³ est un triradical alkyle ;
   quand n = 4 , R³ est un tetraradical alkyle ;
E est -O-, ou -N(R⁴)- ;
R⁴ est hydrogène, un radical alkyle en C₁-C₅ ou hydroxyalkyle en C₁-C₅.

Comme radicaux alkyle en C₁-C₂₀, on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme radicaux cycloalkyles en C₃-C₁₀, on peut citer par exemple : cyclopropyle, cyclobutyle, cyclopentyle, cyclohexyle, cycloheptyle, 1-méthylcyclopropyle, 1-éthylcyclopropyle, 1-propylcyclopropyle, 1-butylcyclopropyle, 1-pentylcyclopropyle, 1-méthyl-1-butylcyclopropyle, 1,2-diméthylcyclypropyle, 1-méthyl-2-éthylcyclopropyle, cyclooctyle, cyclononyle ou cyclodécyle.

Comme radicaux cycloalcènyles en C₃-C₁₀ ayant une ou plusieurs doubles liaisons, on peut citer : cyclopropènyle, cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctatétraènyle, cyclononènyle ou cyclodécènyle.

Comme exemples de cycle à 5 ou 6 chaînons formé par les radicaux R¹ et R² avec l'atome d'azote, on peut citer en particulier la pyrrolidine ou la pipéridine.

Un composé de formule (I) avec n = 1 tout particulièrement préféré est le 2-(4-diéthylamino-2-hydroxybenzoyl)-benzoate de n-hexyle de formule (a) : 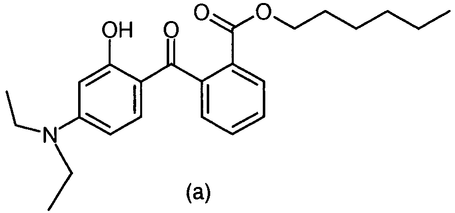 produit vendu sous le nom commercial « UVINUL A+ » par la société BASF.

Un composé de formule (1) avec n = 2 tout particulièrement préféré est le dérivé (2-{4-[2-(4-diethylamino-2-hydroxy-benzoyl)-benzoyl]-piperazine-1-carbonyl}-phenyl)-(4-diethylamino-2-hydroxy-phenyl)-methanone de formule (b) : 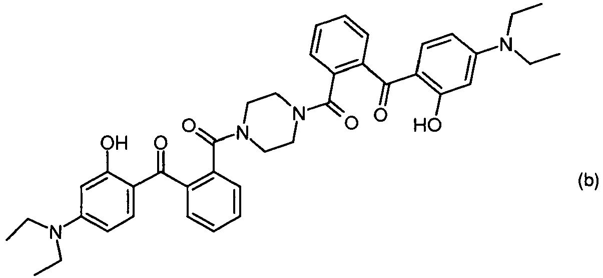 produit décrit dans la demande de brevet WO 2007071584.

Les composés de formule (I) tels que définis ci-dessus sont connus en eux-mêmes et leurs structures et leurs synthèses sont décrites dans les demandes de brevet EP-A-1046391, EP1133980, DE10012408 et WO 2007071584.

Le ou les filtres liphophiles 2-hydroxybenzophénone peuvent être présents dans les compositions conformes à l'invention à des teneurs qui varient de préférence de 0,1 à 10% en poids et plus préférentiellement de 0,1 à 6% en poids par rapport au poids total de la composition.

### FILTRES S-TRIAZINES SILICIES

Les composés s-triazines siliciés conformes à la présente invention répondent à la formule générale (III) suivante ou l'une de ses formes tautomères : 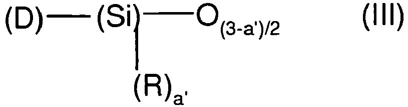 dans laquelle
- R, identiques ou différents représentent un radical alkyle en C₁-C₂, un radical phényle, un radical alkoxy en C₁-C₂, un radical hydroxy ou le groupe triméthylsilyloxy ;
- a' = 1 à 3 ;
- le groupe (D) désigne un composé s-triazine de formule (IV) suivante : 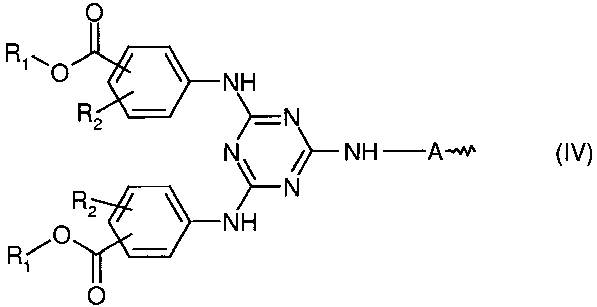 où
   - Ri, identiques ou différents, représentent un radical alkyle en C₁-C₁₀, linéaire ou ramifié et éventuellement insaturé et pouvant contenir un groupe cycloalkyle en C₅-C₆,
   - le groupement (C=O)OR₁ pouvant être en position ortho, méta ou para du groupement amino,
   - R₂, identiques ou différents, représentent hydrogène, un radical hydroxy, un radical alkyle en C₁-C₄, linéaire ou ramifié, un radical méthoxy,
   - A est un radical divalent choisi parmi méthylène ou un groupe répondant à l'une des formules (V), (VI) , (VII) ou (VIII) suivantes :

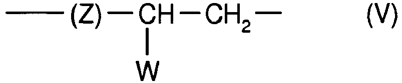

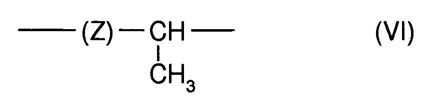

      -(Z)-CH=CH- (VII)

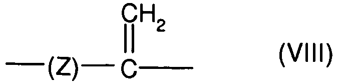

      dans lesquelles :
      - Z est un diradical alkylène en C₁-C₃,
      - W représente un atome d'hydrogène, un radical hydroxyle ou un radical alkyle en C₁-C₃.
en plus des unités de formule -A-(Si)(R)_{a'}(O)_{(3-a')/2}, l'organosiloxane peut comporter des unités de formule : (R)_{b}-(Si)(O)_{(4-b)/2} dans lesquelles : R a la même signification que dans la formule (III), b = 1, 2 ou 3.

Il est à noter que les dérivés de formule (III) peuvent être utilisés sous leurs formes tautomères et plus particulièrement sous la forme tautomère de formule (III') suivante : 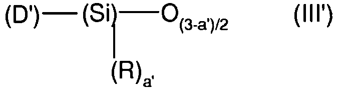 dans laquelle le groupe (D') désigne un composé s-triazine de formule (IV') suivante :

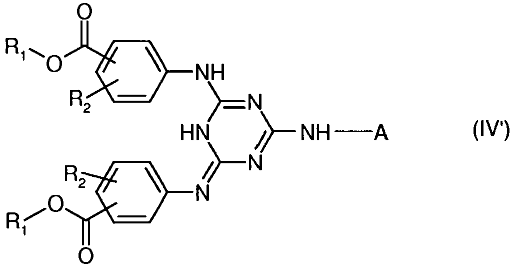

Dans les formules (III) et (III') telles que définies ci-dessus, les radicaux alkyles peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et tert-octyle. Le radical alkyle particulièrement préféré est le radical butyle.

Les dérivés de s-triazine préférentiels sont ceux pour lesquels dans la formule (III) ou (III') au moins l'une, et encore plus préférentiellement l'ensemble, des caractéristiques suivantes sont remplies :
R est méthyle,
a' = 1 ou 2,
R₁ est un radical en C₄-C₈ ,
Z = -CH₂- ,
W=H.

De manière préférée, les composés s-triazine de l'invention sont représentés par les formules (IIIa) ou (IIIb) suivantes : 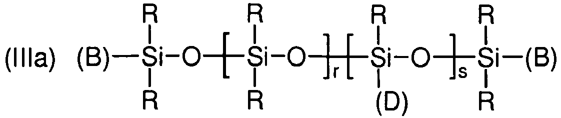 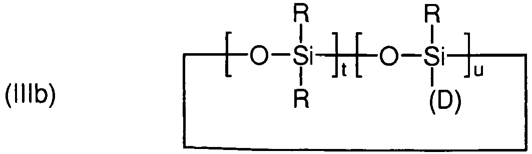 dans lesquelles :
- (D) répond à la formule (III) telle que définie ci-dessus,
- R a la même définition que dans la formule (II),
- (B), identiques ou différents sont choisis parmi les radicaux R et le radical (D),
- r est un nombre entier compris entre 0 et 20 inclusivement,
- s est un nombre entier allant de 0 à 5 et si s = 0, au moins l'un des deux symboles (B) désigne (D),
- u est un nombre entier allant de 1 à 5,
- t est un nombre entier allant de 0 à 5, étant entendu que t + u est égal ou supérieur à 3 ainsi que leurs formes tautomères.

Les diorganosiloxanes linéaires de formule (IIIa) sont particulièrement préférés.

Les diorganosiloxanes linéaires ou cycliques de formule (IIIa) ou (IIIb) rentrant dans le cadre de la présente invention, sont des oligomères statistiques présentant de préférence au moins l'une, et encore plus préférentiellement l'ensemble, des caractéristiques suivantes :
- R est le radical méthyle, le radical alcoxy en C₁-C₂ ou le radical hydroxy,
- B est préférentiellement méthyle (cas des composés linéaires de formule (IIIa)),

A titre d'exemples de composés de formule (I) particulièrement préférés, on citera les composés de formules (1) à (22) suivantes ainsi que leurs formes tautomères :

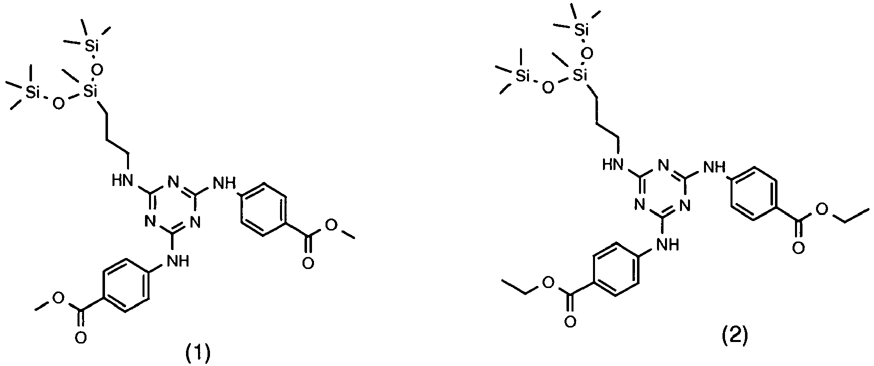

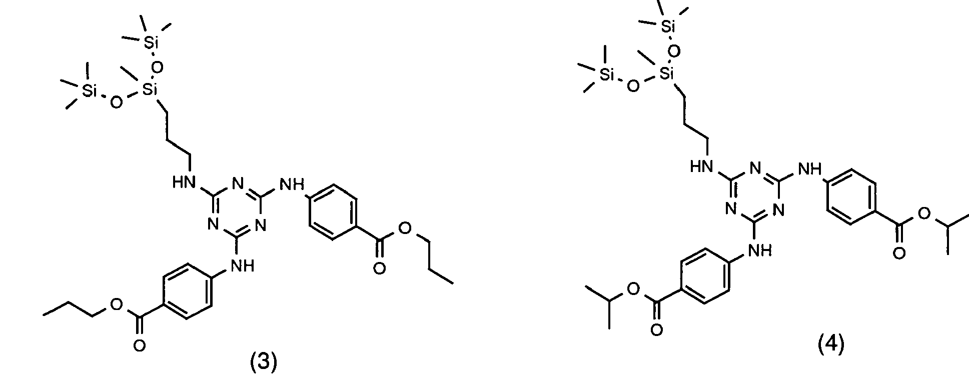

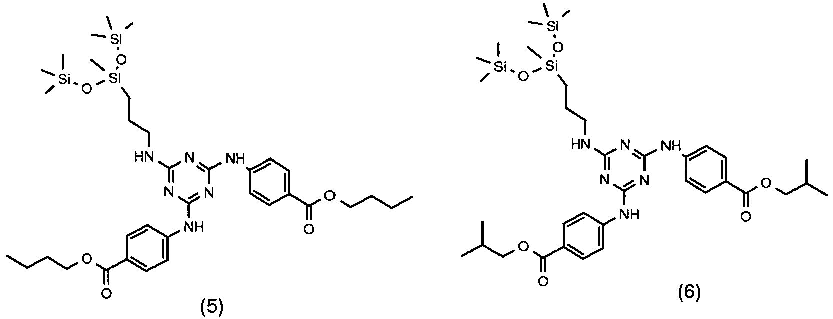

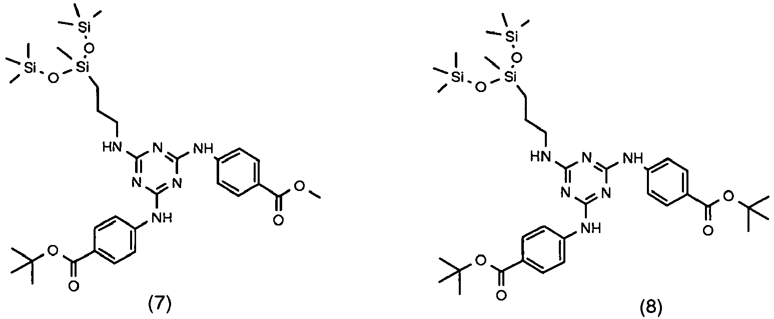

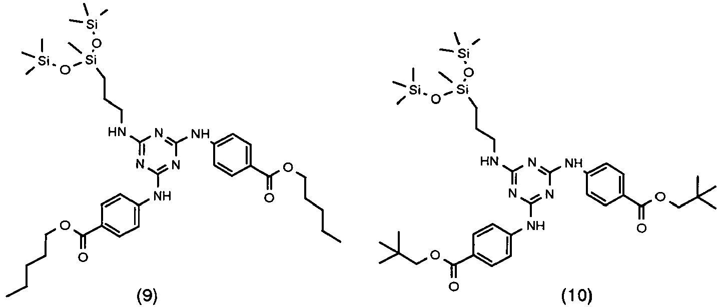

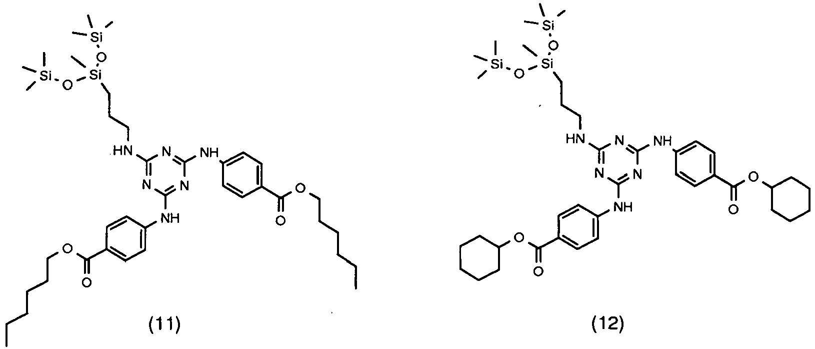

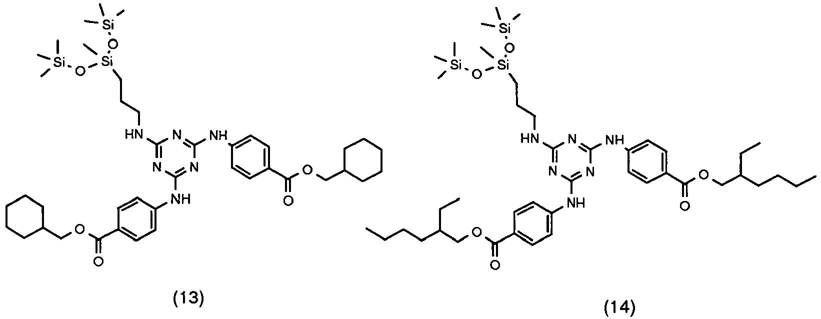

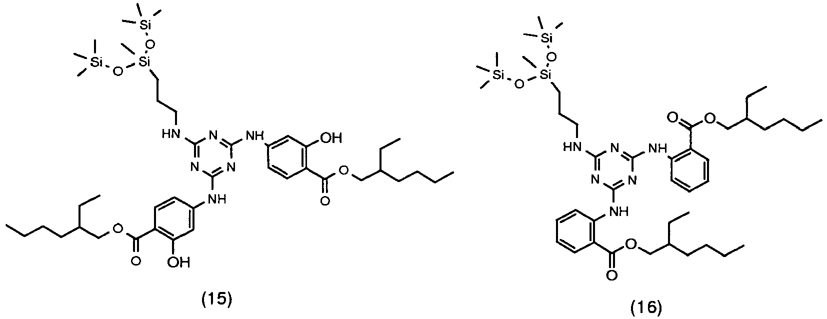

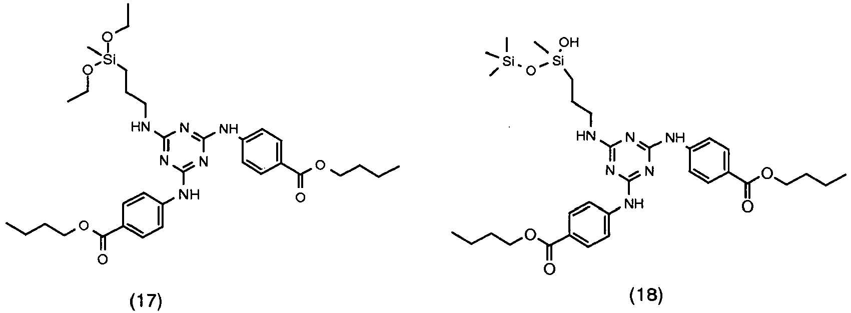

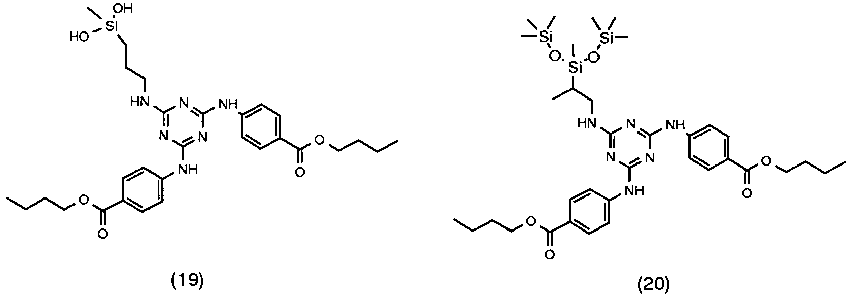

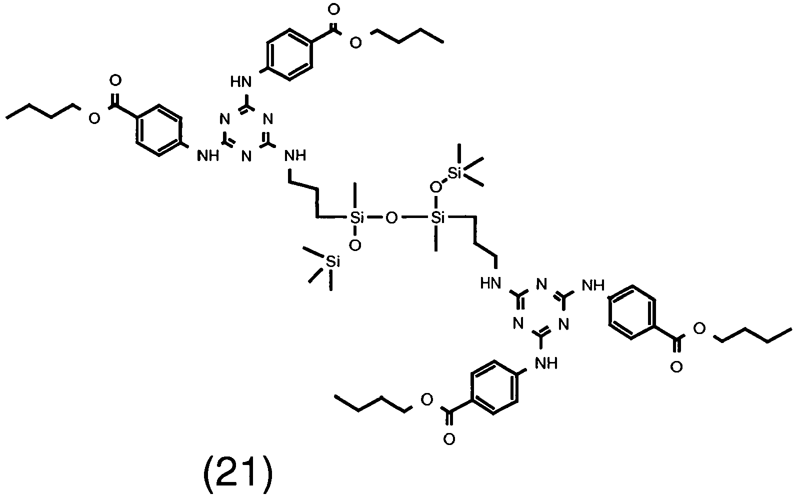

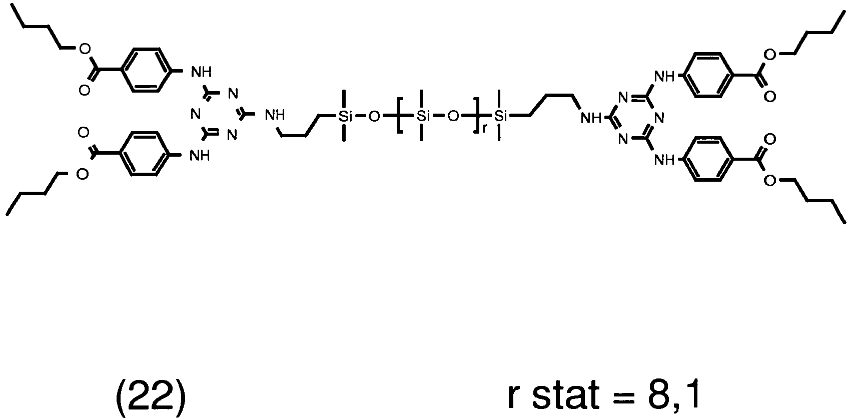

On utilisera plus particulièrement les composés tels le 2,4-bis(4'-aminobenzoate d'éthyle)-6-[(3-{1,3,3,3-tetramethyl-1-[(trimethylsilyl)oxy]disiloxanyl}propyl)amino]-s-triazine de formule (2), le 2,4-bis(4'-aminobenzoate d'isopropyle)-6-[(3-{1,3,3,3-tetramethyl-1-[(trimethylsilyl)oxy]disiloxanyl}propyl)amino]-s-triazine de formule (4), le 2,4-bis(4'-aminobenzoate de n-butyle)-6-[(3-{1,3,3,3-tetramethyl-1-[(trimethylsilyl)oxy]disiloxanyl}propyl)amino]-s-triazine de formule (5), le 2,4-bis(4'-aminobenzoate d'isobutyle)-6-[(3-{1,3,3,3-tetramethyl-1-[(trimethylsilyl)oxy]disiloxanyl}propyl)amino]-s-triazine de formule (6) et le 2,4-bis(4'-aminobenzoate de néopentyle)- 6-[(3-{1,3,3,3-tetramethyl-1-[(trimethylsilyl)oxy]disiloxanyl}propyl)amino]-s-triazine de formule (10).

On utilisera encore plus particulièrement la 2,4-bis(4'-aminobenzoate de n-butyle)-6-[(3-{1,3,3,3-tetramethyl-1-[(trimethylsilyl)oxy]disiloxanyl}propyl)amino]-s-triazine de formule (5) :

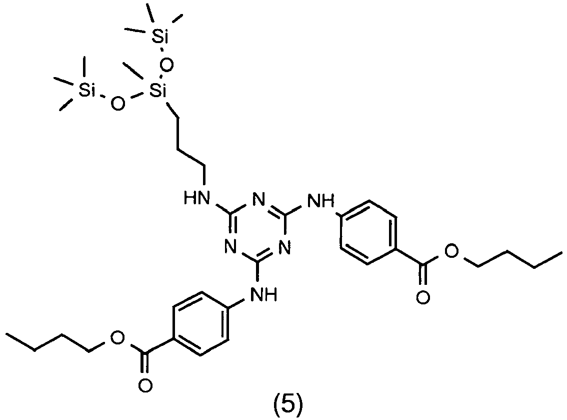

Parmi les composés de formule (III) et leurs formes tautomères, certains sont connus et ont été décrits dans les brevets EP 0841341 et FR 2886143. Parmi les composés de formule (III) et leur formes tautomères, les produits de formules (1), (3), (4), (7), (8), (9), (10), (11), (12) et (13) sont nouveaux.

Les dérivés de triazine de formule (III) conformes à l'invention sont de préférence présents dans les compositions conformes à l'invention à des teneurs de 0,01 à 20% en poids et plus préférentiellement de 0,1 à 10 % et encore plus préférentiellement de 0,1 à 6% en poids par rapport au poids total de la composition.

L'invention se rapporte également à une composition comprenant dans un support cosmétiquement acceptable au moins un système filtrant UV, caractérisée par le fait qu'elle comprend :
(i) au moins un dérivé du dibenzoylméthane ;
(ii) au moins un filtre UV de type 2-hydroxybenzophénone lipophile ; et
(iii) au moins une s-triazine siliciée substituée par au moins deux groupes alkylaminobenzoates de formule (III).

Elle concerne également un procédé de photostabilisation vis-à-vis du rayonnement d'au moins un filtre du type dérivé du dibenzoylméthane par une quantité efficace d'au moins au moins un filtre de type 2-hydroxybenzophénone lipophile et d'au moins une s-triazine siliciée substituée par au moins deux groupements aminobenzoates ou aminobenzamides de formule (III) définie ci-dessous.

### DERIVES DE DIBENZOYLMETHANE

Parmi les dérivés du dibenzoylméthane, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert-butyl-4'-méthxydibenzoylméthane,
- le 2,4-diméthyl-4'-méthoxydibenzoylméthane,
- le 2,6-diméthyl-4-tert-butyl-4'-méthoxydibenzoylméthane.

Parmi les dérivés du dibenzoylméthane mentionnés ci-dessus, on utilisera tout particulièrement le 4-(ter.-butyl) 4'-méthoxy dibenzoylméthane ou Butyl Methoxy Dibenzoylmethane, proposé à la vente sous la dénomination commerciale de "PARSOL 1789" par la Société DSM NUTRITIONAL PRODUCTS ; ce filtre répond à la formule suivante :

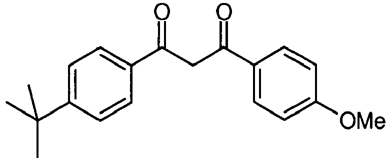

Le ou les dérivés du dibenzoylméthane peuvent être présents dans les compositions conformes à l'invention à des teneurs qui varient de préférence de 0,01 à 10% en poids et plus préférentiellement de 0,1 à 6% en poids par rapport au poids total de la composition.

Les compositions conformes à l'invention peuvent comporter en plus d'autres filtres UV organiques ou inorganiques complémentaires actifs dans l'UVA et/ou l'UVB hydrosolubles ou liposolubles ou bien insolubles dans les solvants cosmétiques couramment utilisés.

Bien entendu, l'homme de l'art veillera à choisir le ou les éventuels filtres complémentaires et/ou leurs quantités de manière telle que les propriétés avantageuses attachées intrinsèquement aux compositions conformes à l'invention ne soient pas, ou substantiellement pas, altérées par la ou les adjonctions envisagées.

Les filtres organiques complémentaires sont notamment choisis parmi les anthranilates ; les dérivés cinnamiques ; les dérivés salicyliques, les dérivés du camphre ; les dérivés de β,β-diphénylacrylate ; les dérivés de triazine autres que ceux de formule (III) ; les dérivés de benzotriazole ; les dérivés de benzalmalonate notamment ceux cités dans le brevet US5624663 ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981.

Comme exemples de filtres UV organiques, on peut citer ceux désignés ci-dessous sous leur nom INCI :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par

### SYMRISE,

Dipropyleneglycol Salicylate vendu sous le nom « DIPSAL » par SCHER,
TEA Salicylate, vendu sous le nom « NEO HELIOPAN TS » par SYMRISE,

### Dérivés cinnamiques :

Ethylhexyl Methoxycinnamate vendu notamment sous le nom commercial
« PARSOL MCX » par DSM NUTRITIONAL PRODUCTS,
Isopropyl Methoxy cinnamate,
Isoamyl Methoxy cinnamate vendu sous le nom commercial « NEO HELIOPAN E 1000 » par SYMRISE,
Cinoxate,
DEA Methoxycinnamate,
Diisopropyl Methylcinnamate,
Glyceryl Ethylhexanoate Dimethoxycinnamate

### Dérivés de β,β-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par

### MERCK,

Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,

### Dérivés du phenyl benzotriazole :

Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés de triazine :

Bis-Ethylhexyloxyphenol Methoxyphenyl Triazine vendu sous le nom commercial «TINOSORB S » par CIBA GEIGY,
Ethylhexyl triazone vendu notamment sous le nom commercial «UVINUL T150 » par BASF,
Diethylhexyl Butamido Triazone vendu sous le nom commercial « UVASORB HEB » par SIGMA 3V,
2,4,6-tris-(4'-amino benzalmalonate de diisobutyle)-s- triazine,
2,4,6-tris(4'-amino benzalmalonate de dinéopentyle)-s-triazine,
2,4-bis(4'-amino benzalmalonate de dinéopentyle)-6-(4'-aminobenzoate de n-butyle)-s-triazine
les filtres triazines symétriques décrits dans le brevet US6,225,467, la demande WO2004/085412 (voir composés 6 et 9) ou le document « Symetrical Triazine Derivatives » IP.COM Journal , IP.COM INC WEST HENRIETTA, NY, US (20 septembre 2004) notamment les 2,4,6-tris-(biphényl)-1,3,5-triazines (en particulier la 2,4,6-tris(biphenyl-4-yl-1,3,5-triazine) et la 2,4,6-tris(terphenyl)-1,3,5-triazine qui est repris dans les demandes de Beiersdorf WO06/035000, WO06/034982, WO06/034991, WO06/035007, WO2006/034992, WO2006/034985.

### Dérivés anthraniliques :

Menthyl anthranilate vendu sous le nom commercial commercial « NEO HELIOPAN MA » par SYMRISE,

### Dérivés d'imidazolines :

Ethylhexyl Dimethoxybenzylidene Dioxoimidazoline Propionate,

### Dérivés du benzalmalonate :

Di-néopentyl 4'-méthoxybenzalmalonate
Polyorganosiloxane à fonctions benzalmalonate comme le Polysilicone-15 vendu sous la dénomination commerciale « PARSOL SLX » par DSM NUTRITIONAL PRODUCTS

### Dérivés de 4.4-diarylbutadiène :

1,1-dicarboxy (2,2'-diméthyl-propyl)-4,4-diphénylbutadiène

### Dérivés de benzoxazole :

2,4-bis-[5-1(diméthylpropyl)benzoxazol-2-yl-(4-phenyl)-imino]-6-(2-ethylhexyl)-imino-1,3,5-triazine vendu sous le nom d'Uvasorb K2A par Sigma 3V
et leurs mélanges.

Les filtres organiques complémentaires préférentiels sont choisis parmi
Ethylhexyl Methoxycinnamate
Homosalate
Ethylhexyl Salicylate,
Octocrylene,
Phenylbenzimidazole Sulfonic Acid,
4-Methylbenzylidene camphor,
Ethylhexyl triazone,
Bis-Ethylhexyloxyphenol Methoxyphenyl Triazine
Diethylhexyl Butamido Triazone,
2,4,6-tris(biphenyl-4-yl-1,3,5-triazine
2,4,6-tris(4'-amino benzalmalonate de dinéopentyle)-s-triazine
2,4,6-tris-(4'-amino benzalmalonate de diisobutyle)-s- triazine,
2,4-bis(4'-amino benzalmalonate de dinéopentyle)-6-(4'-aminobenzoate de n-butyle)-s-triazine
Methylène bis-Benzotriazolyl Tetramethylbutylphénol,
Polysilicone-15
Di-néopentyl 4'-méthoxybenzalmalonate
1,1-dicarboxy (2,2'-diméthyl-propyl)-4,4-diphénylbutadiène 2,4-bis-[5-1(diméthylpropyl)benzoxazol-2-yl-(4-phenyl)-imino]-6-(2-ethylhexyl)-imino-1,3,5-triazine
et leurs mélanges.

Les filtres inorganiques complémentaires sont choisis parmi des pigments d'oxydes métalliques enrobés ou non dont la taille moyenne des particules primaires: est préférentiellement comprise entre 5 nm et 100 nm (de préférence entre 10 nm et 50 nm) comme par exemple des pigments d'oxyde de titane (amorphe ou cristallisé sous forme rutile et/ou anatase), de fer, de zinc, de zirconium ou de cérium qui sont tous des agents photoprotecteurs UV bien connus en soi.

Les pigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

De façon connue, les silicones sont des polymères ou oligomères organo-siliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium.

Le terme "silicones" englobe également les silanes nécessaires à leur préparation, en particulier, les alkyl silanes.

Les silicones utilisées pour l'enrobage des pigments convenant à la présente invention sont de préférence choisies dans le groupe contenant les alkyl silanes, les polydialkylsiloxanes, et les polyalkylhydrogénosiloxanes. Plus préférentiellement encore, les silicones sont choisies dans le groupe contenant l'octyl triméthyl silane, les polydiméthylsiloxanes et les polyméthylhydro-génosiloxanes.

Bien entendu, les pigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Les pigments enrobés sont plus particulièrement des oxydes de titane enrobés :
- de silice tels que le produit "SUNVEIL" de la société IKEDA et le produit " Eusolex T-AVO" de la société MERCK
- de silice et d'oxyde de fer tels que le produit "SUNVEIL F" de la société IKEDA,
- de silice et d'alumine tels que les produits "MICROTITANIUM DIOXIDE MT 500 SA" et "MICROTITANIUM DIOXIDE MT 100 SA" de la société TAYCA, "TIOVEIL" de la société TIOXIDE, et « Mirasun TiW 60 » de la société Rhodia,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que le produit "MICROTITANIUM DIOXIDE MT 100 TV, MT 100 TX, MT 100 Z, MT-01 de la société TAYCA, les produits "Solaveil CT-10 W", "Solaveil CT 100" et "Solaveil CT 200" de la société UNIQEMA,
- de silice, d'alumine et d'acide alginique tel que le produit " MT-100 AQ" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS"de la société TAYCA,
- de silice, d'alumine, de stéarate d'aluminium et traités par une silicone tels que le produit "STT-30-DS" de la société TITAN KOGYO,
- de silice et traité par une silicone tel que le produit "UV-TITAN X 195" de la société KEMIRA, ou le produit SMT-100 WRS de la société TAYCA.
- d'alumine et traités par une silicone tels que les produits "TIPAQUE TTO-55 (S)" de la société ISHIHARA, ou "UV TITAN M 262" de la société KEMIRA, de triéthanolamine tels que le produit "STT-65-S" de la société TITAN KOGYO,
- d'acide stéarique tels que le produit "TIPAQUE TTO-55 (C)" de la société ISHIHARA,
- d'hexamétaphosphate de sodium tels que le produit "MICROTITANIUM DIOXIDE MT 150 W" de la société TAYCA.

D'autres pigments d'oxyde de titane traités avec une silicone sont de préférence le TiO₂ traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO2 traité par un polydiméthylsiloxane et dont la taille moyenne des particules élémentaires est de 21 nm tel que celui vendu sous la dénomination commerciale "70250 Cardre UF TiO2SI3" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les pigments d'oxyde de zinc non enrobés, sont par exemple
- ceux commercialisés sous la dénomination "Z-cote" par la société Sunsmart ;
- ceux commercialisés sous la dénomination "Nanox" par la société Elementis ;
- ceux commercialisés sous la dénomination "Nanogard WCD 2025" par la société Nanophase Technologies ;

Les pigments d'oxyde de zinc enrobés sont par exemple
- ceux commercialisés sous la dénomination « Z-COTE HP1 » par la société SUNSMART (ZnO enrobé dimethicone) ;
- ceux commercialisés sous la dénomination "Oxide zinc CS-5" par la société Toshibi (ZnO enrobé par polymethylhydrogenesiloxane) ;
- ceux commercialisés sous la dénomination "Nanogard Zinc Oxide FN" par la société Nanophase Technologies (en dispersion à 40% dans le Finsolv TN, benzoate d'alcools en C12-C15) ;
- ceux commercialisés sous la dénomination "DAITOPERSION ZN-30" et "DAITOPERSION Zn-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD Ultrafine ZnO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "Escalol Z100" par la société ISP (ZnO traité alumine et dispersé dans le mélange methoxycinnamate d'ethylhexyle / copolymère PVP-hexadecene / methicone) ;
- ceux commercialisés sous la dénomination "Fuji ZnO-SMS-10" par la société Fuji Pigment (ZnO enrobé silice et polymethylsilsesquioxane) ;
- ceux commercialisés sous la dénomination "Nanox Gel TN" par la société Elementis (ZnO dispersé à 55% dans du benzoate d'alcools en C12-C15 avec polycondensat d'acide hydroxystéarique).

Les pigments d'oxyde de cérium non enrobé sont vendus par exemple sous la dénomination "COLLOIDAL CERIUM OXIDE" par la société RHONE POULENC.

Les pigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les pigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Les filtres UV additionnels sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions aqueuses conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire autre que les cires apolaires telles que définies précédemment ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les amides grasses (comme l'isopropyl lauroyl sarcosinate vendu sous la dénomination d' « Eldew SL-205 » par la société Ajinomoto), les acides ou les esters gras comme le benzoate d'alcools en C₁₂-C₁₅ vendu sous la dénomination commerciale « Finsolv TN » ou « Witconol TN » par la société WITCO, le Benzoate de 2-éthylphenyle comme le produit commercial vendu sous le nom X-TEND 226 ® par la société ISP, le palmitate d'octyle, le lanolate d'isopropyle, les triglycérides dont ceux des acides caprique/caprylique, le dicaprylyl carbonate vendu sous la dénomination « Cetiol CC » par la société Cognis), les esters et éthers gras oxyéthylénés ou oxypropylénés; les huiles siliconées (cyclométhicone, polydiméthysiloxanes ou PDMS) ou fluorées, les polyalkylènes, les trimellitates de trialkyle comme le trimellitate de tridécyle.

Comme composés cireux, on peut citer la cire de carnauba, la cire d'abeille, l'huile de ricin hydrogénée, les cires de polyéthylène et les cires de polyméthylène comme celle vendue sous la dénomination Cirebelle 303 par la société SASOL.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le caprylylg glycol, le pentylène glycol, le dipropylène glycol ou le diéthylène glycol.

Comme épaississants hydrophiles, on peut citer les polymères carboxyvinyliques tels que les Carbopols (Carbomers) et les Pemulen (Copolymère acrylate/C10-C30-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination commerciale « Hostacerin AMPS » (nom CTFA : ammonium polyacryloyldimethyl taurate ou le SIMULGEL 800 commercialisé par la société SEPPIC (nom CTFA : sodium polyacryolyldimethyl taurate / polysorbate 80 / sorbitan oleate) ; les copolymères d'acide 2-acrylamido 2-méthylpropane sulfonique et d'hydroxyethyl acrylate comme le SIMULGEL NS et le SEPINOV EMT 10 commercialisés par la société SEPPIC ; les dérivés cellulosiques tels que l'hydroxyéthylcellulose ; les polysaccharides et notamment les gommes telles que la gomme de Xanthane ;_les dérivés siliconés hydrosolubles ou hydrodispersibles comme les silicones acryliques, les silicones polyéthers et les silicones cationiques et leurs mélanges.

Comme épaississants lipophiles, on peut citer les polymères synthétiques tels que les poly C10-C30 alkyl acrylates vendu sous la dénomination « INTELIMER IPA 13-1 » et « INTELIMER IPA 13-6 » par la société Landec ou encore les argiles modifiées telles que l'hectorite et ses dérivés, comme les produits commercialisés sous les noms de Bentone.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées, notamment l'amélioration de la photostabilité du dérivé de dibenzoylméthane.

Les compositions selon l'invention peuvent être préparées selon les techniques bien connues de l'homme de l'art. Elles peuvent se présenter en particulier sous forme d'émulsion, simple ou complexe (H/E, E/H, H/E/H ou E/H/E) telle qu'une crème, un lait ou d'un gel crème ; sous la forme d'un gel aqueux ; sous la forme d'une lotion. Elles peuvent éventuellement être conditionnées en aérosol et se présenter sous forme de mousse ou de spray.

De préférence, les compositions selon l'invention se présentent sous la forme d'une émulsion huile-dans-eau ou eau-dans huile.

Les procédés d'émulsification pouvant être utilisés sont de type pâle ou hélice, rotor-stator et HHP.

Il est également possible, par HHP (entre 50 et 800b), d'obtenir des dispersions stables avec des tailles de gouttes pouvant descendre jusqu'à 100 nm.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les diméthicone copolyols tels que le mélange de cyclométhicone et de diméthicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90R par la société Goldschmidt et le mélange de cétyl diméthicone copolyol, d'isostéarate de polyglycérole (4 moles) et de laurate d'hexyle vendu sous la dénomination ABIL WE 09 par la société Goldschmidt. On peut y ajouter aussi un ou plusieurs co-émulsionnants, qui, de manière avantageuse, peuvent être choisis dans le groupe comprenant les esters alkylés de polyol. Comme esters alkylés de polyol, on peut citer notamment les esters de polyéthylèneglycol comme le PEG-30 Dipolyhydroxystearate tel que le produit commercialisé sous le nom Arlacel P135 par la société ICI.

Comme esters de glycérol et/ou de sorbitan, on peut citer par exemple l'isostéarate de polyglycérol, tel que le produit commercialisé sous la dénomination Isolan GI 34 par la société Goldschmidt ; l'isostéarate de sorbitan, tel que le produit commercialisé sous la dénomination Arlacel 987 par la société ICI ; l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) comme le mélange PEG-100 Stearate/ Glyceryl Stearate commercialisé par exemple par la société ICI sous la dénomination Arlacel 165 ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition auto-émulsionnante, comme décrit par exemple dans le document WO-A-92/06778.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin, des produits de protection solaire et des produits de maquillage.

Les compositions cosmétiques selon l'invention peuvent par exemple être utilisées comme produit de maquillage.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Les compositions selon l'invention peuvent également comprendre en plus des actifs additionnels cosmétiques et dermatologiques.

Parmi les actifs, on peut citer :
- les vitamines (A, C, E, K, PP...) et leurs dérivés ou précurseurs, seuls ou en mélanges,
- les agents anti-glycation ;
- les agents apaisants,
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants;
- les agents tenseurs,
- les agents matifiants,
- les agents kératolytiques,
- les agents desquamants ;
- les agents hydratants comme par exemple les polyols tels que la glycérine, le butylène glycol, propylène glycol.
- les agents anti-inflammatoires ;
- les agents agissant sur le métabolisme énergétique des cellules,
- les agents répulsifs contre les insectes
- les antagonistes de substances P ou de CRGP.
- les agents anti-chute et/ou repousse des cheveux
- les agents anti-rides.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

L'homme du métier choisira le ou lesdits actifs en fonction de l'effet recherché sur la peau, les cheveux, les cils, les sourcils, les ongles.

La composition pourra comprendre en outre au moins un ingrédient tel que des charges à effet flouteur ou des agents favorisant la coloration naturelle de la peau, destiné à compléter l'effet biologique de ces actifs ou apporter un effet anti-âge immédiat visuel.

Pour le soin et/ou le maquillage des peaux grasses, l'homme du métier choisira de préférence au moins un actif choisi parmi les agents desquamants, agents sébo-régulateurs ou anti-séborrhéiques, les agents astringents.

### AUTRES INGREDIENTS ADDITIONNELS

La composition pourra comprendre en outre au moins un ingrédient additionnel destiné à compléter l'effet biologique de ces actifs ou apporter un effet immédiat visuel ; on peut citer notamment les agents matifiants, les charges à effet flouteur, les agents fluorescents, les agents favorisant la coloration naturellement rosée de la peau et les charges abrasives ou exfoliantes..

Pour complémenter et/ou optimiser les effets conférés par les actifs cosmétiques et/ou dermatologiques cités ci-dessus sur les matières kératiniques, il peut être avantageux d'intégrer dans les compositions de l'invention d'autres ingrédients additionnels.

En particulier, ces ingrédients addditionnels pourront conférer un effet immédiat visuel qui sera relayé par l'effet biologique des actifs cités ci-dessus. Ils pourront également, via une action mécanique (ex : charges abrasvies), amplifier l'effet des actifs biologiques cités ci-dessus.

Ainsi la composition selon l'invention pourra comprendre en outre au moins un agent choisi parmi des agents matifiants, des charges à effet flouteur, des agents favorisant la coloration naturellement rosée de la peau, des charges abrasives ou agents exfoliants, et leurs mélanges.

### Agents matifiants

Par "agent matifiant", on entend des agents destinés à rendre la peau visiblement plus mate, moins brillante.

L'effet matifiant de l'agent et/ou de la composition le contenant peut notamment être évalué à l'aide d'un gonioréflectomètre, en mesurant le rapport R entre la réflexion spéculaire et la réflexion diffuse. Une valeur de R inférieure ou égale à 2 traduit généralement un effet matifiant.

L'agent matifiant pourra notamment être choisi parmi un amidon de riz ou un amidon de maïs nom INCI: ZEA MAYS (CORN) STARCH comme en particulier le produit vendu sous le nom commercial « FARMAL CS 3650 PLUS 036500 » par National Starch, la kaolinite, le talc, un extrait de graines de potiron, des microbilles de cellulose, des fibres végétales, des fibres synthétiques, en particulier de polyamides, des microsphères de copolymères acryliques expansées, des poudres de polyamides, les poudres de silice, les poudres de polytétrafluoroéthylène, les poudres de résine de silicone, les poudres de polymères acryliques, les poudres de cire, les poudres de polyéthylène, les poudres d'organopolysiloxane réticulé élastomère enrobées de résine de silicone, les poudres composites de talc/dioxyde de titane/alumine/silice, les poudres de silicates mixtes amorphes, les particules de silicate et notamment de silicate mixte, et leurs mélanges.

Comme exemples d'agents matifiants, on peut citer notamment :
- l'amidon de riz ou de maïs, en particulier un aluminium starch octenyl succinate commercialisé sous la dénomination Dry Flo® par la société National Starch,
- la kaolinite ;
- les silices ;
- le talc ;
- un extrait de graines de potiron tel que commercialisé sous la dénomination Curbilene® par la société Indena ;
- des microbilles de cellulose telles que décrites dans la demande de brevet EP 1 562 562 ;
- des fibres, telles que des fibres de soie, de coton, de laine, de lin, de cellulose extraites notamment du bois, des légumes ou des algues, de polyamide (Nylon®), de cellulose modifiée, de poly-p-phénylène téréphtamide, en acrylique, de polyoléfine, de verre, de silice, d'aramide, de carbone, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges décrites dans la demande de brevet EP 1 151 742 ;
- des microsphères de copolymères acryliques expansées telles que celles commercialisées par la société EXPANCEL sous les dénominations EXPANCEL 551®,
- des charges à effet optique telles que décrites dans la demande de brevet FR 2 869 796, en particulier :
- les poudres de polyamides (Nylon®), comme par exemple les particules de Nylon 12 du type Orgasol d'Arkema de taille moyenne 10 microns et d'indice de réfraction 1,54,
- les poudres de silice, comme par exemple les Silica beads SB150 de Miyoshi de taille moyenne 5 microns et d'indice de réfraction 1,45,
- les poudres de polytétrafluoroéthylène, comme les PTFE ceridust 9205F de Clariant de taille moyenne 8 microns et d'indice de réfraction 1,36,
- les poudres de résine de silicone comme les Silicon resin Tospearl 145A de GE Silicone de taille moyenne 4,5 microns et d'indice de réfraction 1,41,
- les poudres de copolymères acryliques, notamment de poly(méth)acrylate de méthyle, comme les particules PMMA Jurymer MBI de Nihon Junyoki de taille moyenne 8 microns et d'indice de réfraction 1,49, ou les particules Micropearl M100® et F 80 ED® de la société Matsumoto Yushi-Seiyaku,
- les poudres de cire comme les particules Paraffin wax microease 114S de micropowders de taille moyenne 7 microns et d'indice de réfraction 1,54,
- les poudres de polyéthylène, notamment comprenant au moins un copolymère éthylène/acide acrylique, et en particulier constituées de copolymères éthylène/acide acrylique comme les particules Flobeads EA 209 de Sumitomo (de taille moyenne 10 microns et d'indice de réfraction 1,48),
- les poudres d'organopolysiloxane réticulé élastomère enrobées de résine de silicone, notamment de résine silsesquioxane, comme décrit par exemple dans le brevet US 5 538 793. De telles poudres d'élastomère sont vendues sous les dénominations
   "KSP-100", "KSP-101", "KSP-102", "KSP-103", "KSP-104", "KSP-105" par la société SHIN ETSU, et
- les poudres composites de talc/dioxyde de titane/alumine/silice comme celles vendues sous la dénomination Coverleaf® AR-80 par la société Catalyst & chemicals,
- leurs mélanges,
- des composés absorbant et/ou adsorbant le sébum tels que décrits dans la demande de brevet FR 2 869 796. On peut citer notamment :
- les poudres de silice, comme par exemple les microsphères de silice poreuses vendues sous la dénomination "SILICA BEADS SB-700" commercialisées par la société MYOSHI, les "SUNSPHERE® H51", "SUNSPHERE® H33" , "SUNSPHERE® H53" commercialisées par la société ASAHI GLASS ; les microsphères de silice amorphe enrobées de polydiméthylsiloxane vendues sous la dénomination "SA SUNSPHERE® H-33" et "SA SUNSPHERE® H-53" commercialisées par la société ASAHI GLASS ;
- les poudres de silicates mixtes amorphes, notamment d'aluminium et de magnésium, comme par exemple celle commercialisée sous la dénomination "NEUSILIN UFL2" par la société Sumitomo.
- les poudres de polyamides (nylon®), comme par exemple "I'ORGASOL® 4000" commercialisé par la société Arkema, et
- les poudres de polymères acryliques, notamment de polyméthacrylate de méthyle, comme par exemple le "COVABEAD® LH85" commercialisé par la société WACKHERR ; de polyméthacrylate de méthyle/diméthacrylate d'éthylène glycol, comme par exemple le "DOW CORNING 5640 MICROSPONGE® SKIN OIL ADSORBER" commercialisé par la société DOW CORNING, ou le "GANZPEARL® GMP-0820" commercialisé par la société GANZ CHEMICAL ; de polyméthacrylate d'allyle/diméthacrylate d'éthylène glycol, comme par exemple le "POLY-PORE® L200" ou le "POLY-PORE® E200" commercialisés par la société AMCOL ; de copolymère diméthacrylate d'éthylène glycol/méthacrylate de lauryle, comme par exemple le "POLYTRAP® 6603" commercialisé de la société DOW CORNING ;
- les particules de silicate, telle que la silicate d'alumine ;
- les particules de silicates mixtes, telles que :
- les particules de silicate d'aluminium et de magnésium, telles que la saponite ou silicate de magnésium et d'aluminium hydraté avec un sulfate de sodium commercialisée sous la dénomination commerciale Sumecton® par la société Kunimine ;
- le complexe silicate de magnésium, hydroxyéthylcellulose, huile de cumin noir, huile de courge et phospholipides ou Matipure® de Lucas Meyer, et
- leurs mélanges.
Comme agents matifiants préférés, on pourra utiliser selon l'invention un extrait de graines de potiron, un amidon de riz ou de mais, la kaolinite, des silices, le talc, les poudres de polyamides, les poudres de polyéthylènes, les poudres de copolymères acryliques, les microsphères de copolymères acryliques expansées, les microbilles de résines de silicones, les particules de silicate mixte et leurs mélanges.

### Charges à effet flouteur

Ces charges peuvent être tout matériau susceptible de modifier les rides par ses propriétés physiques intrinsèques et de les masquer. Ces charges peuvent notamment modifier les rides par un effet tenseur, un effet de camouflage, ou un effet de floutage.

En tant que charge, on peut donner à titre d'exemples les composés suivants :
- les microparticules poreuses de silice comme par exemple les Silica Beads® SB 150 et SB 700 de Myochi de taille moyenne de 5µm et les SUNSPHERES® série H d'Asahi Glass comme les H33, H51 de taille respectivement de 3,5 et 5 µm.
- les particules hémisphériques creuses de résines de silicones comme les NLK 500®, NLK 506® et NLK 510® de Takemoto Oil and Fat, notamment décrites dans EP-A-1579849,
- les poudres de résine de silicone comme par exemple les SILICON Resin Tospearl® 145 A DE GE silicone de taille moyenne de 4,5µm.
- les poudres de copolymères acryliques, notamment de poly(meth)acrylate de méthyle comme par exemple les particules PMMA Jurimer MBI® de Nihon Junyoki de taille moyenne de 8µm, les sphères creuses de PMMA vendues sous la dénomination COVABEAD® LH 85 par la société Wackherr et les microsphères de vinylidène/acrylonitrile/méthacrylates de méthylène expansées vendues sous la dénomination Expancel®.
- les poudres de cires comme les particules Paraffin wax microloase® 114S de Micropowders de taille moyenne de 7µm.
- les poudres de polyéthylènes notamment comprenant au moins un copolymère éthylène/acide acrylique comme par exemple les FLOBEADS® EA 209 E de Sumimoto de taille moyenne de 10µm.
- les poudres d'organopolysiloxanes élastomériques réticulées enrobées de résine de silicone notamment de silsesquioxane sous la dénomination KSP 100®, KSP 101®, KSP 102®, KSP 103®, KSP 104® et KSP 105® par la société Shin Etsu.
- les poudres composites de talc/dioxyde ou de titane/alumine/silice comme par exemple les Coverleaf AR 80® de la société Catalyst & Chemical.
- le talc, le mica, le kaolin, la lauryl glycine, les poudres d'amidon réticulés par l'anhydride octéanyl succinate, le nitrure de bore, les poudres de polytétrafluoroéthylène, le carbonate de calcium précipité, le carbonate de l'hydrocarbonate de magnésium, le sulfate de baryum, l'hydroxyapatite, le silicate de calcium, le dioxyde de cérium et les microcapsules de verre ou de céramiques.
- les fibres hydrophiles ou hydrophobes synthétiques ou naturelles, minérales ou organiques telles que des fibres de soie, de coton, de laine, de lin, de cellulose extraites notamment du bois, des légumes ou des algues, de polyamide (Nylon®), de cellulose modifiée, de poly-p-phénylène téréphtamide, en acrylique, de polyoléfine, de verre, de silice, d'aramide, de carbone, de polytétrafluoroéthylène (Téflon®), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges décrites dans la demande de brevet EP 1 151 742.
- les silicones réticulés élastomères sphériques comme comme les Trefil E-505C® ou E-506 C® de chez Dow Corning.
- les charges abrasives qui par effet mécanique apportent un lissage du microrelief cutané, telles que la silice abrasive comme par exemple Abrasif SP® de Semanez ou des poudres de noix ou de coques (abricot, noix par exemple de Cosmétochem).

Les charges ayant un effet sur les signes du vieillissement sont notamment choisies parmi des microparticules poreuse de silice, des particules hémisphériques creuses de silicones, des poudres de résine de silicone, des poudres de copolymères acryliques, des poudres de polyéthylènes, des poudres d'organopolysiloxanes élastomériques réticulées enrobées de résine de silicone, des poudres composites de talc/dioxyde de titane/alumine/silice, le carbonate de calcium précipité, le carbonate de l'hydrocarbonate de magnésium, le sulfate de baryum, l'hydroxyapatite, le silicate de calcium, le dioxyde de cérium et les microcapsules de verre ou de céramiques, les fibres de soie, de coton, et leurs mélanges.

La charge peut être une charge « soft focus ».

Par charge « soft-focus », on entend une charge qui en plus donne de la transparence au teint et un effet flou. De préférence, les charges « soft-focus » ont une taille moyenne des particules inférieure ou égale à 15 microns. Ces particules peuvent être de toutes formes et en particulier être sphériques ou non sphériques. De préférence encore, ces charges sont non sphériques.

Les charges « soft-focus » peuvent être choisies parmi les poudres de silice et silicates, notamment d'alumine, les poudres de type polyméthyl méthacrylate (PMMA), le talc, les composites silice/TiO₂ ou silice/oxyde de zinc, les poudres de polyéthylène, les poudres d'amidon, les poudres de polyamides, les poudres de copolymères styrène/acrylique, les élastomères de silicone, et leurs mélanges.

En particulier, on peut citer le talc de taille moyenne en nombre inférieure ou égale à 3 microns, par exemple du talc de taille moyenne en nombre de 1,8 micron et notamment celui vendu sous la dénomination commerciale Talc P3® par la société Nippon Talc, la poudre de Nylon® 12, notamment celle vendue sous le dénomination Orgasol 2002 Extra D Nat Cos® par la société Atochem, les particules de silice traitées en surface par une cire minérale 1 à 2 % (nom INCI : hydrated silica (and) paraffin) telles que celles commercialisées par la société Degussa, les microsphères de silice amorphe, telles que celles vendues sous la dénomination Sunsphère par exemple de référence H-53® par la société Asahi Glass, et les micro-billes de silice telles que celles vendues sous la dénomination SB-700® ou SB-150® par la société Miyoshi, cette liste n'étant pas limitative.

La concentration de ces charges ayant un effet sur les signes du vieillissement dans les compositions selon l'invention peut être comprise entre 0,1 et 40 %, voire entre 0,1 et 20 % en poids par rapport au poids total de la composition.

### Agents favorisant la coloration naturellement rosée de la peau

On peut citer notamment :
- un agent autobronzant, c'est-à-dire un agent qui, appliqué sur la peau, notamment sur le visage, permet d'obtenir un effet de bronzage d'apparence plus ou moins semblable à celui qui peut résulter d'une exposition prolongée au soleil (bronzage naturel) ou sous une lampe UV ;
- un agent de coloration additionnel, c'est-à-dire tout composé ayant une affinité particulière pour la peau lui permettant de conférer à cette dernière une coloration durable, non-couvrante (à savoir n'ayant pas tendance à opacifier la peau) et qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un pigment de maquillage ; et leurs mélanges.

Comme exemples d'agents autobronzants, on peut citer notamment :
la dihydoxyacétone (DHA),
l'érythrulose, et
l'association d'un système catalytique formé de :
sels et oxydes de manganèse et/ou de zinc, et
hydrogénocarbonates alcalins et/ou alcalinoterreux.

Les agents autobronzants sont généralement choisis parmi les composés mono ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342. On utilisera de préférence la DHA.

La DHA peut être utilisée sous forme libre et/ou encapsulée par exemple dans des vésicules lipidiques telle que des liposomes, notamment décrits dans la demande WO 97/25970.

D'une manière générale, l'autobronzant est présent en une quantité allant de 0,01 à 20 % en poids, et de préférence en quantité comprise entre 0,1 et 10 % du poids total de la composition.

On peut encore utiliser d'autres colorants qui permettent de modifier la couleur produite par l'agent autobronzant.

Ces colorants peuvent être choisis parmi les colorants directs synthétiques ou naturels.

Ces colorants peuvent être choisis par exemple parmi les colorants rouges ou oranges du type fluorane tels que ceux décrits dans la demande de brevet FR2840806. On peut citer par exemple les colorants suivants :
- le tetrabromofluoroscéine ou éosine connue sous le nom CTFA : CI 45380 ou Red 21
- la phloxine B connue sous le nom CTFA : CI 45410 ou Red 27
- la diiodofluorescéine connue sous le nom CTFA CI 45425 ou Orange 10 ;
- la dibromofluorescéine connue sous le nom CTFA : Cl 45370 ou Orange 5.
- le sel de sodium de la tetrabromofluoroscéine connue sous le nom CTFA : Cl 45380 (Na salt) ou Red 22
- le sel de sodium de la phloxine B connu sous le nom CTFA : CI 45410 (Na salt) ou Red 28
- le sel de sodium de la diiodofluorescéine connu sous le nom CTFA : CI 45425 (Na salt) ou Orange 11 ;
- l'érythrosine connu sous le nom CTFA : CI 45430 ou Acid Red 51.
- la phloxine connu sous le nom CTFA : CI 45405 ou Acid Red 98.

Ces colorants peuvent être également choisis parmi les antraquinones , le caramel, le carmin, le noir de charbon, les bleus azulènes, le methoxalène, le trioxalène, le guajazulène, le chamuzulène, le rose de bengale, la cosine 10B, la cyanosine, la daphinine.

Ces colorants peuvent être également choisis parmi les dérivés indoliques comme les monohydroxyindoles tels que décrits dans le brevet FR2651126 ( ie : 4-, 5-, 6- ou 7-hydroxyindole) ou les di-hydroxyindoles tels que décrits dans le brevet EP-B-0425324 (ie : 5,6-dihydroxyindole, 2-méthyl 5,6-dihydroxyindole, 3-méthyl 5,6-dihydroxyindole, 2,3-diméthyl 5,6-dihydroxyindole) ;

### Charges abrasives ou agents exfoliants

Comme agents exfoliants utilisables dans des compositions rincées selon l'invention, on peut citer par exemple des particules exfoliantes ou gommantes d'origine minérale, végétale ou organique. Ainsi, on peut utiliser par exemple des billes ou de la poudre de polyéthylène, de la poudre de nylon, de la poudre de polychlorure de vinyle, de la pierre ponce, des broyats de noyaux d'abricots ou de coques de noix, de la sciure de bois, des billes de verre, l'alumine, et leurs mélanges. On peut citer aussi l'Exfogreen® de Solabia (extrait de bambou), des extraits d'akenes de fraises (Akenes de fraise de Greentech), de la poudre de noyau de pêche, la poudre de noyau d'abricot, et enfin dans le domaine des poudres végétales à effet abrasif, citons la poudre de noyaux d'airelles (cranberry).

Comme charges abrasives ou agents exfoliants préférés selon l'invention, on citera la poudre de noyaux de pêche, la poudre de noyaux d'abricot, la poudre de noyaux d'airelles, les extraits d'akènes de fraise, les extraits de bambou.

Les exemples qui suivent servent à illustrer l'invention sans toutefois présenter un caractère limitatif. Dans ces exemples, les quantités des ingrédients compositions sont données en % pondéral par rapport au poids total de la composition.

### I/ EXEMPLES DE SYNTHESE DES TRIAZINES DE FORMULE (III) :

### EXEMPLE 1 : Préparation du 2,4-bis(4'-diylamino benzoate de méthyle)-6-{[1,3,3,3-tétraméthyl-1-[(triméthylsilyl)oxy]disiloxanyl]propyl-3-ylamino}-s-triazine (Voie 1) :

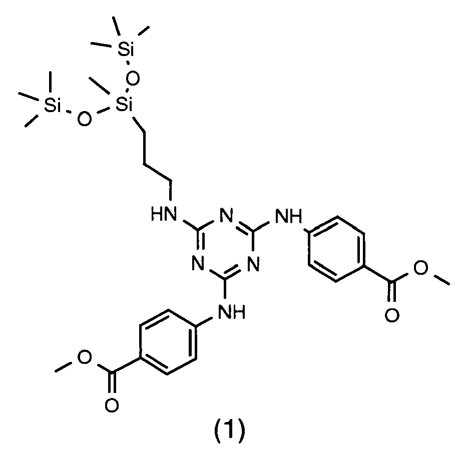

Dans un réacteur sous bullage d'azote, on introduit successivement le para-aminobenzoate de méthyle (9,2 g, 0,0609 mole), la 2-butanone (40 ml) et le carbonate de potassium (4,21 g) dissout dans 57 ml d'eau. On refroidit à 0-5°C et introduit goutte à goutte en 1 heure le chlorure de cyanuryle (5,61 g, 0,0304 mole) dissout dans 88 ml de 2-butanone. On chauffe à 70°C pendant 6 heures ; le 2,4-bis-(4'-diylamino benzoate de méthyle)-6-chloro-s-triazine formé n'est pas isolé.

Au mélange réactionnel, on ajoute 2,56 g de bicarbonate de sodium, puis on coule goutte à goutte en 1 heure l'amino-1[1,3,3,3-tétraméthyl-1-[(triméthylsilyl)oxy]disiloxanyl]-3-propane (8,51 g, 0,0304 mole). Après refroidissement, on sépare les 2 phases et lave la phase organique avec de l'eau et la sèche. On élimine la 2-butanone sous pression réduite. La pâte obtenue est purifiée par passage sur colonne de silice (éluant : Heptane/EtOAc 8 :2). On récupère ainsi les fractions propres du dérivé de l'exemple 1 (3,53 g, Rendement : 18%) sous forme d'une poudre beige :
Point fusion : 144-145°C,
UV (Ethanol) : λₘₐₓ = 311 nm , E_{1%} = 1268.

### EXEMPLE 2 : Préparation du 2,4-bis(4'-diylamino benzoate d'éthyle)-6-{[1,3,3,3-tétraméthyl-1-[(triméthylsilyl)oxy]disiloxanyl]propyl-3-ylamino}-s-triazine (Voie 1) :

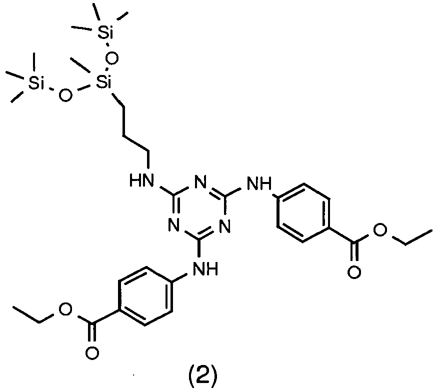

Dans un réacteur sous bullage d'azote, on introduit successivement le para-aminobenzoate d'éthyle (6,05 g, 0,0366 mole), la 2-butanone (30 ml) et le carbonate de potassium (2,53 g) dissout dans 38 ml d'eau. On refroidit à 0-5°C et introduit goutte à goutte en 1 heure le chlorure de cyanuryle (3,38 g, 0,0183 mole) dissout dans 68 ml de 2-butanone. On chauffe à 70°C pendant 6 heures ; la 2,4-bis-(4'-diylamino benzoate d'éthyle)-6-chloro-s-triazine formée n'est pas isolée et est mise en réaction dans l'étape suivante.

Au mélange réactionnel, on ajoute 1,54 g de bicarbonate de sodium, puis on coule goutte à goutte en 1 heure l'amino-1 [1,3,3,3-tétraméthyl-1-[(triméthylsilyl)oxy]disiloxanyl]-3-propane (5,12 g, 0,0183 mole). On maintient à 70°C pendant 4 heures. Après refroidissement, on sépare les 2 phases et lave la phase organique avec de l'eau et la sèche. On élimine la 2-butanone sous pression réduite. La pâte obtenue est cristallisée dans l'heptane. On obtient ainsi le produit de l'exemple 2 (8,8 g, Rendement 70%) sous forme de cristaux beiges clairs :
Point fusion : 134-135°C,
UV (Ethanol) : λ_{maX} = 311 nm , E_{1%} = 1186.

### EXEMPLE 3 : Préparation du 2,4-bis(4'-diylamino benzoate d'éthyle)-6-{(1,3,3,3-tétraméthyl-1-[(triméthylsilyl)oxy]disiloxanyl]propyl-3-ylamino}-s-triazine (Voie 2) :

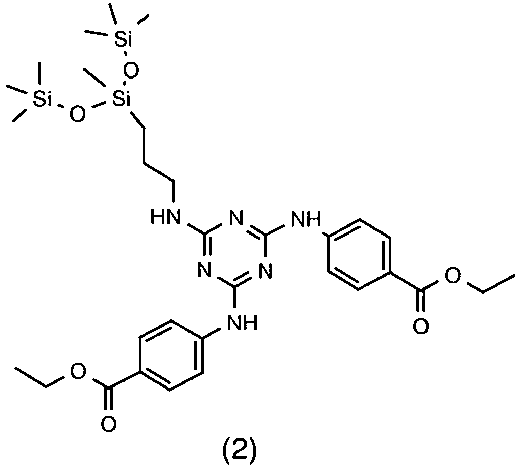

### Première étape : préparation de la 2,4-dichloro-6-{[1,3,3,3-tétraméthyl-1-[(triméthysilyl)oxy]disiloxanyl]propyl-3-ylamino}-s-triazine :

A une solution de chlorure de cyanuryle (25 g, 0,135 mole) dans 250 ml d'acétone, on ajoute goutte à goutte à 0°C l'amino-1[1,3,3,3-tétraméthyl-1-[(triméthylsilyl)oxy]disiloxanyl]-3-propane (41,7 g, 0,149 mole) et une solution de bicarbonate de sodium (11,4 g, 0,135 mole) dans 120 ml d'eau de telle sorte que le pH se situe entre 3 et 6,5. En fin d'introduction, le pH est de 6,5. L'agitation est ensuite maintenue 1 heure 30 minutes à 10°C, puis laissé à température du labo. Le précipité formé est filtré, lavé à l'eau, essoré et séché. On obtient 55,2 g (Rendement : 95%) du dérivé attendu sous forme d'une poudre blanche (Pf : 59°C).

### Deuxième étape : préparation du composé (2):

Le mélange du produit précédent (2,1 g, 0,005 mole) et de para-amino benzoate d'éthyle (1,65 g, 0,01 mole) en suspension dans 20 ml de toluène est chauffé au reflux pendant 1 heure 30 minutes. On refroidit et on ajoute à la résine obtenue de l'heptane chaud. Après trituration, filtration et séchage, on obtient 2,3 g (Rendement : 67%) du dérivé de l'exemple 3 sous forme d'une poudre blanche :
Point fusion : 126-128°C,
UV (Ethanol) : λₘₐₓ = 311 nm , E_{1%} = 1147.

### EXEMPLE 4 : Préparation du 2,4-bis(4'-diylamino benzoate de n-propyle)-6-{[1,3,3,3-tétraméthyl-1-[(triméthylsilyl)oxy]disiloxanyl]propyl-3-ylamino}-s-triazine (Voie 2) :

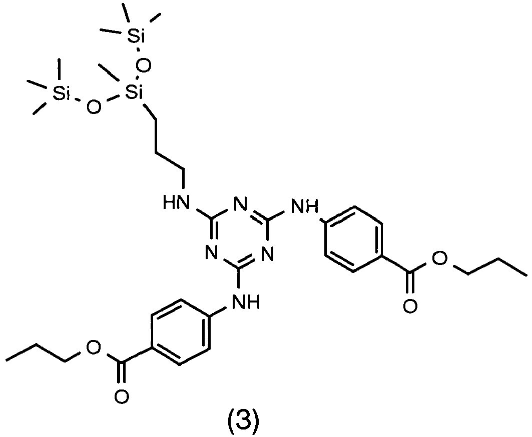

On introduit dans un réacteur le para-aminobenzoate de n-propyle (3,35 g, 0,0187 mole) dissout dans 15 ml d'acétate d'éthyle. On y ajoute 1,5 ml de pyridine dissout dans 10 ml d'acétate d'éthyle. On chauffe le mélange réactionnel à 70°C sous atmosphère d'argon puis on ajoute le produit de la première étape de l'exemple 3 (4 g, 0,0094 mole). On laisse à 70°C pendant 2 heures. La solution rouge foncée est refroidit et est lavée avec des solutions saturée en chlorure de sodium, puis à l'eau. Après séchage de la phase organique, la pâte obtenue est cristallisée dans l'heptane. On obtient ainsi le produit de l'exemple 4 (2,3 g, Rendement : 34%) sous forme de cristaux beige clair :
Point fusion : 109-110°C,
UV (Ethanol) : λₘₐₓ = 311 nm , E_{1%} = 1228.

### EXEMPLE 5 : Préparation du 2,4-bis(4'-diylamino benzoate d'isopropyle)-6-{[1,3,3,3-tétraméthyl-1-[(triméthylsilyl)oxy]disiloxanyl]propyl-3-ylamino}-s-triazine (Voie II) :

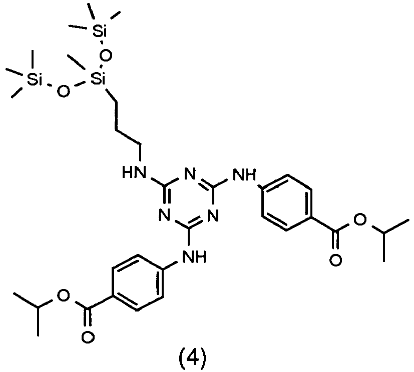

On introduit dans un réacteur le para-aminobenzoate d'isopropyle (10,05 g, 0,0561 mole) dissout dans 40 ml d'acétate d'éthyle. On y ajoute 4,54 ml de pyridine dissout dans 15 ml d'acétate d'éthyle. On chauffe le mélange réactionnel à 70°C sous atmosphère d'Argon puis on ajoute le produit de la première étape de l'exemple 3 (12 g, 0,0281 mole). On laisse à 70°C pendant 2 heures. La solution rouge foncée est refroidit et est lavée avec des solutions saturée en chlorure de sodium, puis à l'eau. Après séchage de la phase organique, la pâte obtenue est purifiée par chromatographie sur colonne de Silice (éluant : Heptane/EtOAc 8 :2) cristallisée dans l'heptane. On obtient ainsi le produit de l'exemple 5 (10,8 g, Rendement : 54%) sous forme de poudre beige clair :
Point fusion : 68-70°C,
UV (Ethanol) : λₘₐₓ = 311 nm , E_{1%} = 1155.

### EXEMPLE 6 : Préparation du 2,4-bis(4'-diylamino benzoate de n-butyle)-6-{[1,3,3,3-tétraméthyl-1-[(triméthylsilyl)oxy]disiloxanyl]propyl-3-ylamino}-s-triazine (Voie II) :

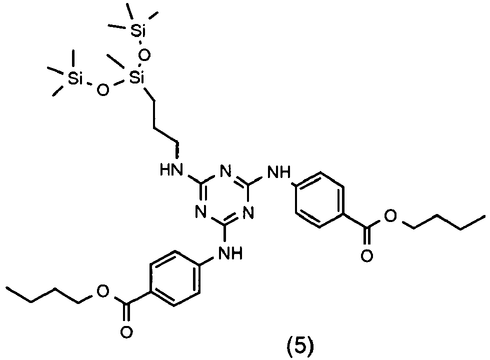

Sous barbotage d'azote, le produit obtenu à la première étape de l'exemple 3 (16,74 g, 0,0391 mole), du para-amino benzoate de n-butyle (15 g, 0,0776 mole) et du carbonate de potassium (5,36 g, 0,0388 mole) sont mis en suspension dans 170 ml de toluène et sont chauffés au reflux pendant 1 heure 20 minutes. On refroidit le mélange réactionnel et on y ajoute 150 ml de dichlorométhane. Les minéraux sont filtrés. Le filtrat est lavé à l'eau bicarbonatée puis 2 fois à l'eau. On obtient après séchage de la phase organique et évaporation des solvants une poudre blanche. Après recristallisation dans un mélange EtOAc/Heptane 1 :15, on obtient 20,1 g (Rendement : 69%) du dérivé de l'exemple 6 sous forme d'une poudre blanche :
Point fusion : 111-113°C,
UV (Ethanol) : λₘₐₓ = 312 nm , E_{1%} = 1360.

### EXEMPLE 7 : Préparation du 2,4-bis(4'-diylamino benzoate d'isobutyle)-6-{[1,3,3,3-tétraméthyl-1-[(triméthylsilyl)oxy]disiloxanyl]propyl-3-ylamino}-s-triazine (Voie 1) :

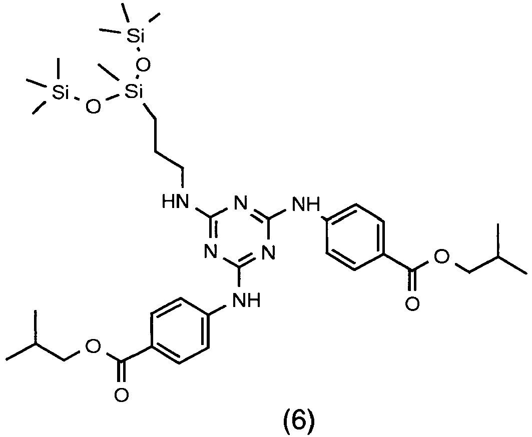

Dans un réacteur sous bullage d'azote, on introduit successivement le para-aminobenzoate d'isobutyle (10,39 g, 0,0538 mole), la 2-butanone (72 ml) et le carbonate de potassium (3,72 g) dissout dans 57 ml d'eau. On refroidit à 0-5°C et introduit goutte à goutte en 1 heure et 30 minutes le chlorure de cyanuryle (4,96 g, 0,0269 mole) dissout dans 68 ml de 2-butanone. On chauffe à 70°C pendant 17 heures ; la 2,4-bis-(4'-diylamino benzoate d'isobutyle)-6-chloro-s-triazine formée n'est pas isolée.

Au mélange réactionnel, on ajoute 2,26 g de bicarbonate de sodium, puis on coule goutte à goutte en 1 heure le produit de la première étape de l'exemple 2 bis (7,52 g, 0,0269 mole). Après refroidissement, on sépare les 2 phases et lave la phase organique avec de l'eau et la sèche. On élimine la 2-butanone sous pression réduite. La pâte obtenue est chromatographiée sur colonne de Silice (éluant : Heptane / EtOAc 8 :2). On obtient ainsi le produit de l'exemple 7 (5,2 g, Rendement 26%) sous forme de cristaux beiges clairs :
Point fusion : 66-67°C,
UV (Ethanol) : λₘₐₓ = 311 nm , E_{1%} = 1150.

### EXEMPLE 8 : Préparation du 2-(4'-ylamino benzoate de méthyle)-4-(4"-ylamino benzoate de tertio-butyle)-6-{[1,3,3,3-tétraméthyl-1-[(triméthylsilyl)oxy]disiloxanyl]propyl-3-ylamino}-s-triazine (Voie 1 modifiée) :

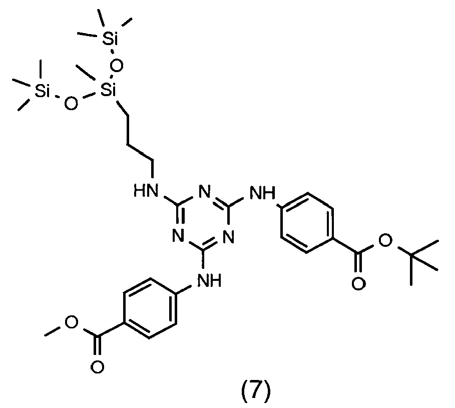

Dans un réacteur sous bullage d'azote, on solubilise le para-aminobenzoate de tert-butyle (1 g, 0,00517 mole) dans 10 ml d'acétate d'éthyle. On y ajoute la pyridine (836 µl) dissoute dans 5 ml d'acétate d'éthyle. On chauffe à 70°C et coule goutte à goutte en 30 minutes une solution du dérivé de la première étape de l'exemple 2 bis (2,21 g, 0,00517 mole) dans 15 ml d'acétate d'éthyle. On laisse à 70°C pendant 2 heures et ajoute ensuite une solution de para-aminobenzoate (0,98 g, 0,00517 mole) dissout dans 5 ml d'acétate d'éthyle. On continue de chauffer à 70°C pendant 2 heures. Après refroidissement, on lave la phase organique avec une solution saturée de chlorure de sodium, puis à l'eau et la sèche. La pâte obtenue est chromatographiée sur colonne de Silice (éluant : Heptane / EtOAc 8 :2). On obtient ainsi le produit de l'exemple 8 (1,52 g, Rendement 30%) sous forme de cristaux beiges clairs :
Point fusion : 111-113°C,
UV (Ethanol) : λₘₐₓ = 311 nm, E_{1%} = 1425.

### EXEMPLE 9 : Préparation du 2,4-bis(4'-diylamino benzoate de tert-butyle)-6-{[1,3,3,3-tétraméthyl-1-[(triméthylsilyl)oxyldisiloxanyl]propyl-3-ylamino}-s-triazine (Voie 2) :

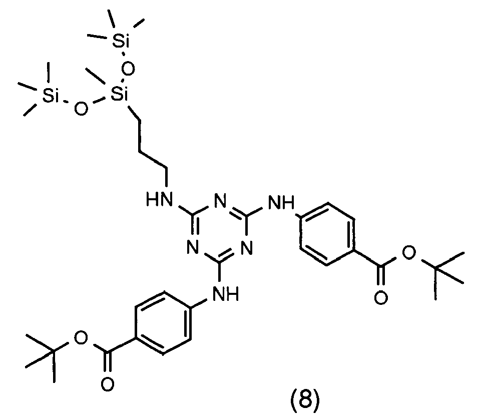

Dans un réacteur sous bullage d'azote, on solubilise le para-aminobenzoate de tert-butyle (3,62 g, 0,00189 mole) dans 15 ml d'acétate d'éthyle. On y ajoute la pyridine (1,5 ml) dissoute dans 10 ml d'acétate d'éthyle. On chauffe à 70°C et coule goutte à goutte en 30 minutes une solution du dérivé de la première étape de l'exemple 3 (4 g, 0,00936 mole) dans 15 ml d'acétate d'éthyle. On laisse à 70°C pendant 2 heures. Après refroidissement, on lave la phase organique avec une solution saturée de chlorure de sodium, puis à l'eau et la sèche. La pâte obtenue est chromatographiée sur colonne de Silice (éluant : Heptane / EtOAc 8 :2). On obtient ainsi les fractions propres du produit de l'exemple 9 (3,94 g, Rendement 42%) sous forme d'une poudre blanche :
Point fusion : 78-79°C,
UV (Ethanol) : λₘₐₓ = 311 nm , E_{1%} = 1321.

### EXEMPLE 10 : Préparation du 2,4-bis(4'-diylamino benzoate de n-pentyle)-6-{[1,3,3,3-tétraméthyl-1-[(triméthylsilyl)oxy]disiloxanyl]propyl-3-lamino}-s-triazine (Voie 1) :

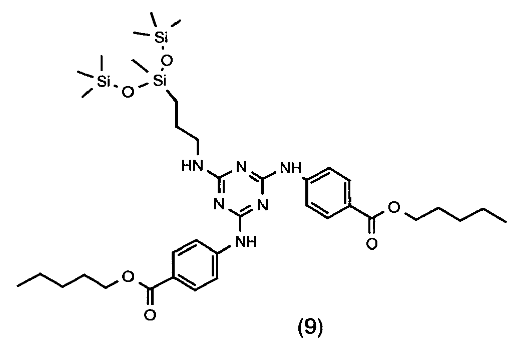

Dans un réacteur sous bullage d'azote, on introduit successivement le para-aminobenzoate de n-pentyle (40 g, 0,0193 mole), la 2-butanone (200 ml) et le carbonate de potassium (13,34 g) dissout dans 116 ml d'eau. On refroidit à 0-5°C et introduit goutte à goutte en 1 heure et 30 minutes le chlorure de cyanuryle (17,8 g, 0,0965 mole) dissout dans 68 ml de 2-butanone. On chauffe à 70°C pendant 17 heures ; la 2,4-bis-(4'-diylamino benzoate de n-penttyle)-6-chloro-s-triazine formée n'est pas isolée.
Au mélange réactionnel, on ajoute 8,11 g de bicarbonate de sodium, puis on coule goutte à goutte en 1 heure le produit de la première étape de l'exemple 2 bis (27 g, 0,00965 mole). On maintient le chauffage à 70°C pendant 4 heures. Après refroidissement, on sépare les 2 phases et lave la phase organique avec de l'eau et la sèche. On élimine la 2-butanone sous pression réduite. Le solide jaune orangé obtenu est chromatographiée sur colonne de Silice (éluant : Heptane / EtOAc 9 :1). On obtient ainsi le produit de l'exemple 10 (3,79 g, Rendement 51%) sous forme de cristaux beiges clairs :
Point fusion : 94-95°C,
UV (Ethanol) : λₘₐₓ = 311 nm , E_{1%} = 1265.

### EXEMPLE 11 : Préparation du 2,4-bis(4'-diylamino benzoate de n-hexyle)-6-{[1,3,3,3-tétraméthyl-1-[(triméthylsilyl)oxy]disiloxanyl]propyl-3-ylamino}-s-triazine (Voie 2) :

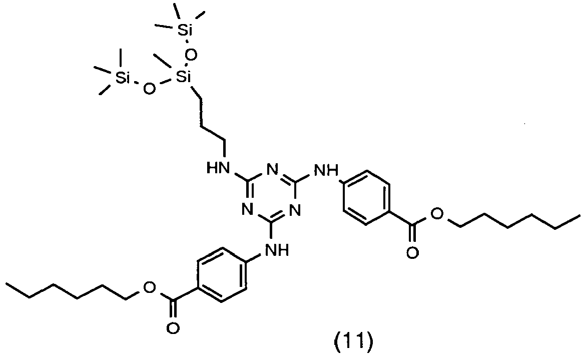

Dans un réacteur sous bullage d'argon, on solubilise le para-aminobenzoate de n-hexyle (19,32 g, 0,0904 mole) dans 50 ml d'acétate d'éthyle. On y ajoute la pyridine (7,3 ml) dissoute dans 15 ml d'acétate d'éthyle. On chauffe à 70°C et coule goutte à goutte en 30 minutes une solution du dérivé de la première étape de l'exemple 3 (19,32 g, 0,0452 mole) dans 50 ml d'acétate d'éthyle. On laisse à 70°C pendant 2 heures. Après refroidissement, on lave la phase organique avec une solution saturée de chlorure de sodium, puis à l'eau et la sèche. La pâte obtenue est chromatographiée sur colonne de Silice (éluant : Heptane / EtOAc 9 :1). On obtient ainsi les fractions propres du produit de l'exemple 11 (12,4 g, Rendement 34%) sous forme d'une poudre blanche :
Point fusion : 40-41 °C,
UV (Ethanol) : λₘₐx = 311 nm, E_{1%} = 1371.

### EXEMPLE 12 : Préparation du 2,4-bis(4'-diylamino benzoate de cyclohexyle)-6-{[1,3,3,3-tétraméthyl-1-[(triméthylsilyl)oxy]disiloxanyl]propyl-3-ylamino}-s-triazine (Voie II) :

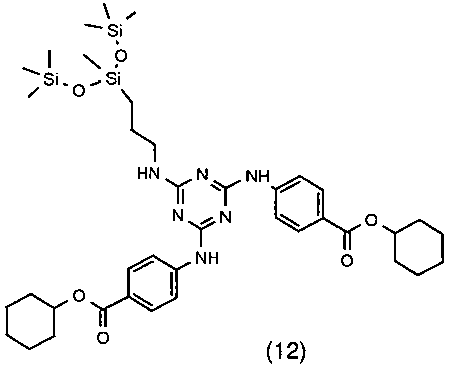

Dans un réacteur sous bullage d'argon, on solubilise le para-aminobenzoate de cyclohexyle (20 g, 0,0912 mole) dans 50 ml d'acétate d'éthyle. On y ajoute la pyridine (7,4 ml) dissoute dans 15 ml d'acétate d'éthyle. On chauffe à 70°C et coule goutte à goutte en 30 minutes une solution du dérivé de la première étape de l'exemple 3 (19,5 g, 0,0456 mole) dans 50 ml d'acétate d'éthyle. On laisse à 70°C pendant 2 heures. Après refroidissement, on lave la phase organique avec une solution saturée de chlorure de sodium, puis à l'eau et la sèche. La pâte obtenue est chromatographiée sur colonne de Silice (éluant : Heptane / EtOAc 8 :2). On obtient ainsi les fractions propres du produit de l'exemple 12 (31,8 g, Rendement 82%) sous forme d'une poudre beige :
Point de fusion : 74-75°C,
UV (Ethanol) : λₘₐₓ = 311 nm, E_{1%} = 1412.

### EXEMPLE 13 : Préparation du 2,4-bis(4'-diylamino benzoate de cyclohexyle)-6-{[1,3,3,3-tétraméthyl-1-[(triméthylsilyl)oxy]disiloxanyl]propyl-3-ylamino}-s-triazine (Voie II) :

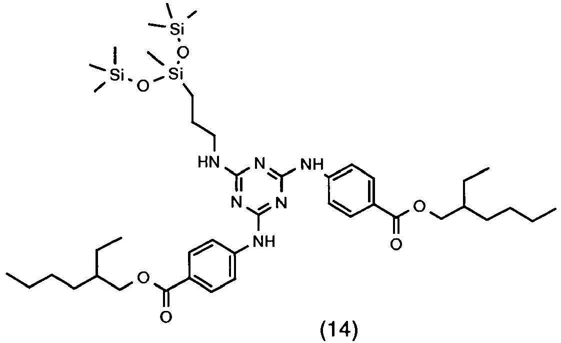

Dans un réacteur sous bullage d'argon, on solubilise le para-aminobenzoate de 2-éthyl hexyle (20 g, 0,0802 mole) dans 50 ml d'acétate d'éthyle. On y ajoute la pyridine (6,5 ml) dissoute dans 15 ml d'acétate d'éthyle. On chauffe à 70°C et coule goutte à goutte en 30 minutes une solution du dérivé de la première étape de l'exemple 3 (17,2 g, 0,0401 mole) dans 50 ml d'acétate d'éthyle. On laisse à 70°C pendant 2 heures. Après refroidissement, on lave la phase organique avec une solution saturée de chlorure de sodium, puis à l'eau et la sèche. La pâte obtenue est chromatographiée sur colonne de Silice (éluant : Heptane / EtOAc 8 :2). On obtient ainsi les fractions propres du produit de l'exemple 13 (14,4 g, Rendement 42%) sous forme d'une cire marron pâle :
UV (Ethanol) : λₘₐₓ = 311 nm , E_{1%} = 1450.

### EXEMPLE 14 : Préparation du 2,4-bis(2'-hydroxy-4'-diylamino benzoate d'éthyl-2-hexyle)-6-{[1,3,3,3-tétraméthyl-1-[(triméthylsilyl)oxy]disiloxanyl]propyl-3-ylamino}-s-triazine (Voie II) :

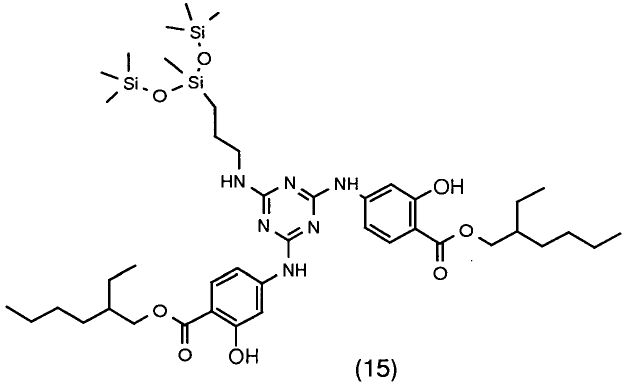

Sous barbotage d'azote, un mélange de 2-hydroxy-4-aminobenzoate d'éthyl-2-hexyle (1,4 g, 5,57x10-3 mole) et du produit de la première étape de l'exemple 3 (1,19 g, 2,78x10-3 mole) dans 10 ml de toluène est chauffé au reflux pendant 5 heures. On refroidit et on évapore le solvant. Le résidu est chromatographié sur colonne de silice (éluant : Heptane/EtOAc 9/1). On obtient 1,58 g (Rendement : 64%) des fractions propres du dérivé de l'exemple 14 sous forme d'une pâte blanche :
UV (Ethanol) :
   λₘₐₓ = 300 nm , E_{1%} = 480
   λₘₐₓ = 325 nm, E_{1%} = 709.

### EXEMPLE 15 : Préparation du butyl 4-{[4-{[4-(butoxycarbonyl)phenyl]amino}-6-({3-[diethoxy(methyl)silyl]propyl}amino)-1,3,5-triazin-2-yl]amino}benzoate (Voie II) :

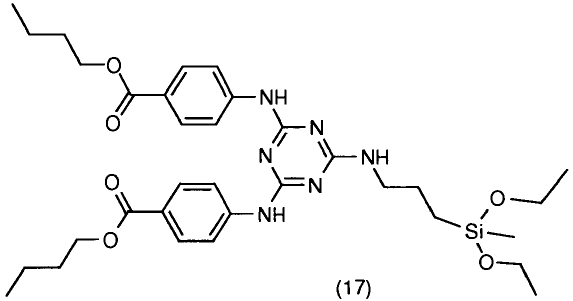

### Première étape : préparation du 2,4-bis-(4'-diylamino benzoate de n-butyle)-6-chloro-s-triazine :

A une solution de chlorure de cyanuryle (54,36 g, 0,295 mole) dans 500 ml de dioxane et 50 ml d'eau, on ajoute goutte à goutte à 5°C simultanément du para-amino benzoate de n-butyle (113,94 g, 0,59 mole) et une solution de carbonate de potassium (40,68 g, 0,295 mole) dans 50 ml d'eau de telle sorte que le pH se situe entre 3 et 6,5. On maintient pendant 1 heure 30 minutes à 5°C. Un précipité se forme dans le milieu qui correspond à la s-triazine monosubstituée. On chauffe progressivement à 70°C et ajoute le deuxième équivalent de carbonate de potassium (40,68 g, 0,295 mole) dans 50 ml d'eau. L'agitation est ensuite maintenue 5 heures à 70°C. On refroidit et on filtre le mélange réactionnel. Le précipité formé est lavé à l'eau, essoré et séché. Après recristallisation dans du dioxane/eau, on obtient après séchage sous vide 52,5 g (Rendement : 36%) du premier jet de recristallisation de 2,4-bis-(4'-diylamino benzoate de n-butyle)-6-chloro-s-triazine sous forme d'une poudre blanche.

Sous barbotage d'azote, le mélange hétérogène du produit de la première étape de l'exemple (20 g, 0,04 mole) et d'aminopropyl diéthoxy méthyl silane (15,37 g, 0 ,08 mole) est chauffé progressivement jusqu'à 70°C. Au bout d'une heure, on refroidit, on ajoute du dichlorométhane et on lave la phase organique 3 fois à l'eau. On obtient après séchage de la phase organique et évaporation des solvants puis par une recristallisation dans l'heptane 21 g (Rendement 80%) d'un solide blanc du dérivé de l'exemple 15 :
UV (Ethanol) : λₘₐₓ = 311 nm , E_{1%} = 1197.

### EXEMPLES 16 et 17 : Préparation des dérivés : butyl 4-[(4-{[4-(butoxycarbonyl)phenyl]amino}-6-{[3-(1-hydroxy-1,3,3,3-tetramethyldisiloxanyl)propyl]amino}-1,3,5-triazin-2-yl)amino]benzoate et dibutyl 4,4'-{[6-({3-dihydroxy(methyl)silyl]propyl}amino)-1,3,5-triazine-2,4-diyl]diimino}dibenzoate (obtenus par traitement acide du dérivé de l'exemple 5) :

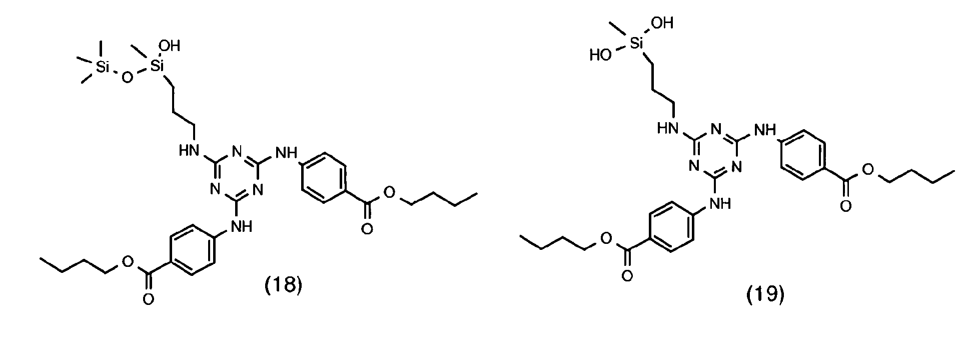

Au dérivé de l'exemple 5 (10 g, 0,013 mole) solubilisé dans 500 ml du mélange éthanol/isopropanol dans le rapport 80:20, on ajoute 160 ml d'acide chlorhydrique 0,1 N et 340 ml du mélange ethanol/isopropanol dans le rapport 80:20. On laisse sous agitation à température du labo pendant 5 heures. Cette solution est neutralisée avec de la soude à 0,4% jusqu'à un pH de 7. On y ajoute 1 litre d'eau et la solution est lyophilisée. Les lots lyophilisés sont rassemblés pour donner 6,5 g d'une poudre beige clair qui contient en pourcentages relatifs par HPLC environ 28% du dérivé de l'exemple 18 et environ 10% du dérivé de l'exemple 19. Cette poudre a été fractionnée par chromatographie de partage centrifuge (avec systèmes biphasiques composés d'heptane, d'acétate d'éthyle, de méthanol et d'eau dans différentes proportions) pour donner 0,58 g du dérivé de l'exemple 16 sous forme de poudre blanche :
UV (Ethanol) : λₘₐₓ = 312 nm , E_{1%} = 1228
et 0,42g du dérivé de l'exemple 17 sous forme d'une poudre blanche.

### EXEMPLE 18 : Préparation du butyl 4-({4-{[4-(butoxycarbonyl)phenyl]amino}-6-[(2-(1,3,3,3-tetramethyl-1-[(trimethylsilyl)oxy]disiloxanyl}propyl)amino]-1,3,5-triazin-2-yl}amino)benzoate :

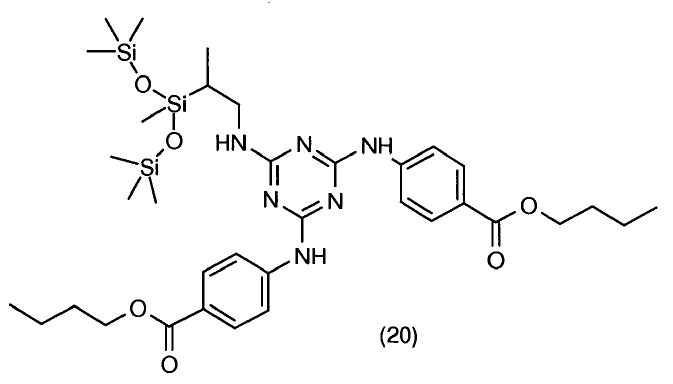

### Première étape : préparation du 4,6-dichloro-N-(2-{1,3,3,3-tetramethyl-1-[(trimethylsily)oxy]disiloxanyl}propyl)-1,3,5-triazin-2-amine :

A une solution de chlorure de cyanuryle (32,5 g, 0,176 mole) dans 180 ml d'acétone, on ajoute goutte à goutte à 0°C un mélange 15/85 de 2-{1,3,3,3-tetramethyl-1-[(trimethylsilyl)oxy]disiloxanyl}propan-1-amine et de 3-{1,3,3,3-tetramethyl-1-[(trimethylsilyl)oxy]disiloxanyl}propan-1-amine (49,3 g, 0,176 mole) et une solution de bicarbonate de sodium (14,8 g, 0,176 mole) dans 210 ml d'eau de telle sorte que le pH se situe entre 4 et 5,8. En fin d'introduction, le pH est de 5,3. L'agitation est ensuite maintenue 1 heure 30 minutes à 10°C, puis laissé à température du labo. Le précipité formé est filtré, lavé à l'eau, essoré et séché. On obtient 72,4 g (Rendement : 96%) des dérivés isomères dans le rapport 15/85 sous forme d'une poudre blanche (Pf : 59°C). 20 g de ce mélange a été fractionné par chromatographie de partage centrifuge (système biphasique : Heptane/Acétonitrile/Eau 50 :49 :1) pour donner 2,0 g de 4,6-dichloro-N-(2-{1,3,3,3-tetramethyl-1-[(trimethylsilyl)oxy]disiloxanyl}propyl)-1,3,5-triazin-2-amine engagé tel quel dans l'étape suivante :

### Deuxième étape : préparation du dérivé de l'exemple 20 :

Le produit précédent (2 g, 0,0047 mole) est solubilisé dans 18 ml de toluène. On y ajoute de la pyridine (0,8 ml, 0,009 mole) et du para-amino benzoate de n-butyle (1,8 g, 0,009 mole). On chauffe à 70°C sous agitation pendant 3 heures. On refroidit et verse la solution sur un lit de Silice et rince le gâteau avec 80 ml de toluène. Après évaporation du solvant, le solide beige marron obtenu est cristallisé dans 30 ml d'heptane. On obtient ainsi 2,2g (Rendement 63%) du dérivé de l'exemple 18 sous forme d'une poudre beige clair :
Point de fusion : 149-151°C,
UV (Ethanol) : λₘₐₓ = 312 nm , E_{1%} = 955.

### EXEMPLE 19 : Préparation du composé (21) :

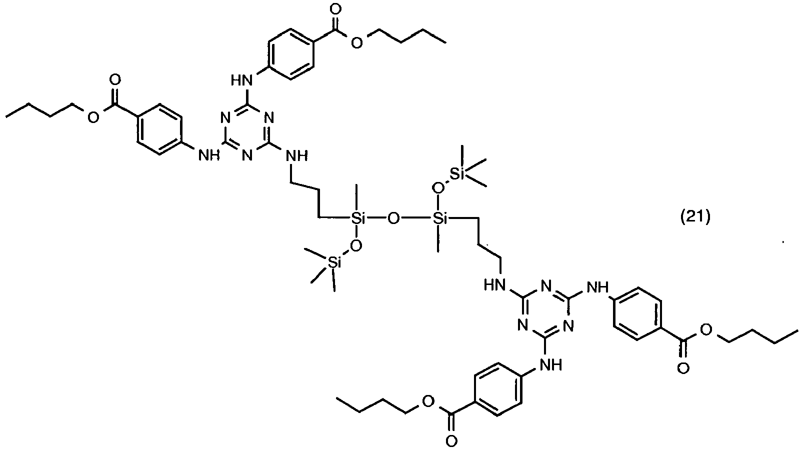

Au dérivé de l'exemple 5 (1 g, 0,0013 mole) solubilisé dans 50 ml du mélange ethanol/isopropanol dans le rapport 80:20, on ajoute 25 ml d'acide chlorhydrique 1N. On laisse sous agitation à température du labo pendant 4 heures. Cette solution est neutralisée avec de la soude à 35% jusqu'à un pH de 7. Les solvants sont évaporés sous vide. On obtient 0,8g d'une poudre beige clair qui contient en pourcentage relatif par HPLC environ 37% du dérivé de l'exemple 21. Cette poudre a été fractionnée par chromatographie de partage centrifuge (avec système biphasique composés d'heptane, d'acétate d'éthyle, de méthanol et d'eau) pour donner 0,18g du dérivé de l'exemple 19 sous forme d'une poudre blanche :
UV (Ethanol) : λₘₐₓ = 312 nm , E_{1%} = 1109.

### EXEMPLE 20 : Préparation du composé (22) :

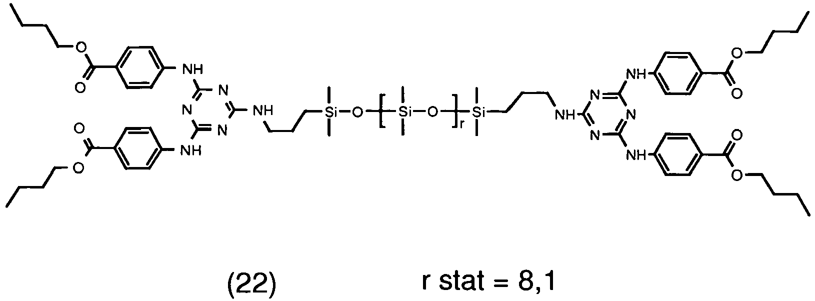

### Première étape : préparation du 2,4-bis(4'-diylamino benzoate de n-butyle)-6-chloro-s-triazine :

A une solution de chlorure de cyanuryle (54,36 g, 0,295 mole) dans 500 ml de dioxane et 50 ml d'eau, on ajoute goutte à goutte à 5°C simultanément du para-amino benzoate de n-butyle (113,94 g, 0,59 mole) et une solution de carbonate de potassium (40,68 g, 0,295 mole) dans 50 ml d'eau de telle sorte que le pH se situe entre 3 et 6,5. On maintient pendant 1 heure 30 minutes à 5°C. Un précipité se forme dans le milieu qui correspond à la s-triazine monosubstituée. On chauffe progressivement à 70°C et ajoute le deuxième équivalent de carbonate de potassium (40,68 g, 0,295 mole) dans 50 ml d'eau. L'agitation est ensuite maintenue 5 heures à 70°C. On refroidit et on filtre le mélange réactionnel. Le précipité formé est lavé à l'eau, essoré et séché. Après recristallisation dans du dioxane/eau, on obtient après séchage sous vide 52,5 g (Rendement : 36%) du premier jet de recristallisation de 2,4-bis-(4'-diylamino benzoate de n-butyle)-6-chloro-s-triazine sous forme d'une poudre blanche.

### Deuxième étape : préparation du dérivé de l'exemple 20 :

Sous barbotage d'azote, un mélange du produit précédent (2 g, 4x10⁻³ mole), d'aminopropyl terminated polydimethylsiloxane (DMS-A-11 de chez Gelest) (2,13 g, 2x10⁻³ mole) et de pyridine (0,32 ml, 4x10-3 mole) dans 40 ml de toluène est chauffé à 70°C pendant 5 heures. On refroidit, on ajoute du dichlorométhane et on lave la phase organique 3 fois à l'eau. On obtient après séchage de la phase organique et évaporation des solvants une huile marron. Après traitement au noir dans l'éthanol à chaud et filtration sur Célite, on obtient 3,3 g (Rendement : 70%) du dérivé de l'exemple 20 sous forme d'une gomme marron clair :
UV (Ethanol) : λₘₐₓ = 311 nm , E_{1%} = 916.

### II/ SYNTHESE DE L' EXEMPLE COMPARATIF (A): Préparation du 2,4-bis(4'-diylamino benzoate de dodecyle)-6-{[1,3,3,3-tétraméthyl-1-[(triméthylsilyl)oxy]disiloxanyl]propyl-3-ylamino}-s-triazine (selon la voie 1+2):

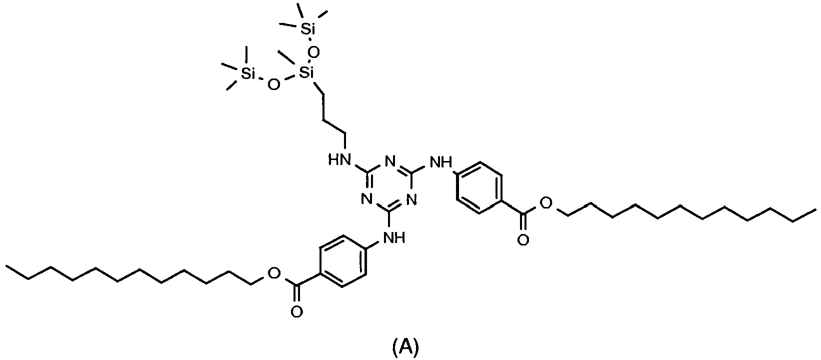

### Première étape : préparation du para-aminobenzoate de dodécyle :

Un mélange de para-aminobenzoate d'éthyle (33,8 g, mole), de n-dodecanol (114,84 ml, mole), de catalyseur (diacétate de dibutyl étain : 544 µL) est chauffé à 170°C pendant 48 heures (l'éthanol formé est éliminé par distillation). Le mélange réactionnel passe de l'incolore à l'orange. Sous un vide d'environ 20 mmHg, le mélange est chauffé 6 heures à 100°C. Le mélange réactionnel passe de l'orangé au rouge. On chauffe ensuite à 120°C pour éliminer l'alcool laurique en excès.
L'huile visqueuse rouge brun est cristallisée dans le méthanol. On obtient après filtration, lavage du gâteau avec du méthanol froid et séchage sous vide le para-aminobenzoate de docécyle (52,77 g, Rendement 84%) sous forme d'une poudre jaune pâle et utilisée telle qu'elle dans l'étape suivante :

### Deuxième étape : préparation du dérivé de l'exemple comparatif A :

Sous balayage d'argon et sous agitation, on ajoute au produit de la première étape de l'exemple 2 bis (20 g, mole) solubilisé dans de l'acétate d'éthyle, 5,3 ml de pyridine en solution dans de l'acétate d'éthyle. On chauffe à 70°C et ajoute par portions en 10 minutes le para-amino benzoate de dodécyle de l'étape précédente (14 g, mole). On laisse le mélange réactionnel à cette température pendant 3 heures. Le mélange réactionnel de couleur rouge/noir est refroidit et est lavé 2 fois avec une solution saturée de chlorure de sodium puis à l'eau en suspension dans 20 ml de toluène est chauffé au reflux pendant 1 heure 30 minutes. On refroidit et ajoute à la résine obtenue de l'heptane chaud. Après trituration, filtration et séchage, on obtient 2,3 g (Rendement : 67%) du dérivé de l'exemple comparatif A sous forme d'une cire marron pâle :
Point de fusion : 134-135°C,
UV (Ethanol) : λₘₐₓ = 311 nm, E_{1%} = 838.

### III/ EXEMPLES DE FORMULATION

Les compositions A (invention) et les compositions hors invention B, C, D, E suivantes ont été réalisées :

| **Ingrédients** | **Ex A** | **ExB (*)** | **ExC (*)** | **Ex D (*)** | **Ex E (*)** |
|---|---|---|---|---|---|
| Phase a | | | | | |
| EDTA | 0,1 | 0,1 | 0,1 | 0,1 | 0,1 |
| Phosphate de mpnocetyle monopotassique | 1 | 1 | 1 | 1 | 1 |
| Eau désionisée | qsp 100 | qsp 100 | qsp 100 | qsp 100 | qsp 100 |
| Triéthanolamine | 0,3 | 0,3 | 0,3 | 0,3 | 0,3 |
| Conservateurs | 1,2 | 1,2 | 1,2 | 1,2 | 1,2 |
| Phase b₁ | | | | | |
| C₁₂-C₁₅ alkyl benzoate | 20 | 20 | 20 | 20 | 20 |
| Conservateurs | 0,25 | 0,25 | 0,25 | 0,25 | 0,25 |
| Acide stéarique | 1,5 | 1,5 | 1,5 | 1,5 | 1,5 |
| Mélange Mono/Distéarate de Glycéryle/ Stéarate de Polyéthylèneglycol (100 OE) | 1 | 1 | 1 | 1 | 1 |
| Alcool cétylique | 0,5 | 0 ,5 | 0,5 | 0,5 | 0,5 |
| Cetearyl alcool et cetearyl glucoside | 2 | 2 | 2 | 2 | 2 |
| Dimethicone (350 cst) | 0,5 | 0,5 | 0,5 | 0,5 | 0,5 |
| Triéthanolamine | 0,45 | 0,45 | 0,45 | 0,45 | 0,45 |
| 2-(4-diéthylamino-2-hydroxybenzoyl)-benzoate de n-hexyle (UVINUL A+) | 4 | 4 | 4 | 4 | 4 |
| s-triazine siliciée substituée par deux C4-alkylaminobenzoates composé (5) | 4 | 0 | 0 | 0 | 0 |
| s-triazine siliciée substituée par deux C₁₂-alkylaminobenzoates Composé de l'exemple (A) | 0 | 4 | 0 | 0 | 0 |
| α-cyano-β,β' diphénylacrylate de 2-éthylhexyle | 0 | 0 | 4 | 0 | 0 |
| 4-méthoxycinnamate d'éthyl-2-hexyle | 0 | 0 | 0 | 4 | 0 |
| 2,4,6-tris[p-(2'-éthylhexyl-1 '-oxy-carbonyl)anilino]-1,3,5-triazine | 0 | 0 | 0 | 0 | 4 |
| Phase b₂ | | | | | |
| Isohexadécane | 1 | 1 | 1 | 1 | 1 |
| Copolymère d'acide acrylique et de méthacrylate d'alkyle en C10-C30 | 0,2 | 0,2 | 0,2 | 0,2 | 0,2 |
| Xanthane | 0,2 | 0,2 | 0,2 | 0,2 | 0,2 |
| Cyclopentadiméthylsiloxane | 5 | 5 | 5 | 5 | 5 |

Ces compositions ont été évaluées selon les propriétés suivantes : Solubilité des filtres, Efficacité filtrante, Photostabilité.

### Mode de préparation des émulsions:

Les phases aqueuse A et huileuse B1 sont préparées par mélange des matières premières sous agitation mécanique à 80°C ; les solutions obtenues sont macroscopiquement homogènes. L'émulsion est préparée par introduction lente de la phase huileuse dans la phase aqueuse sous agitation à l'aide d'un homogénéisateur de type Moritz sous une vitesse d'agitation de 4000 RPM pendant 15 minutes. L'émulsion obtenue est refroidie sous agitation à 40°C, puis y est ajoutée la phase huileuse B2 sous agitation douce. L'émulsion obtenue est refroidie à la température ambiante sous agitation lente. Elle se caractérise par des gouttes de taille comprise entre 1 µm et 10 µm.

### Protocole d'évaluation de la solubilité des filtres UV

Préparation des solutions de filtre UV : Dans un flacon verre de 10ml, on pèse le filtre et l'huile pour un mélange total de 5g. On met en solution à chaud au bain marie à 80°C sous agitation magnétique. Quand la mise en solution est complète (solution limpide), on stocke la solution à température ambiante. On suit l'apparition de cristaux macroscopique dans le temps.

### Protocole d'évaluation in vitro de l'efficacité filtrante

Support d'étalement : PMMA dépoli
Quantité après application : 0.6 mg/cm²
Application : Doigt nu
Temps de séchage : 20 minutes à TA, à l'obscurité
Nombre d'échantillons :
   5 applications par formule
   5 points de mesures par application
Appareil de mesure : Analyseur UV - Labsphère UV 1000S
   Enregistrement des facteurs de protection monochromatique tous les nm entre 290 et 400 nm
   Calcul du FPS selon la méthode décrite par B.L. Diffey. et al. Dans J. Soc. Cosmet. Chem. 40-127-133 (1989).
Spectre de source : Soleil de Diffey 1989
Spectre d'action : Erythème CIE 1987

### Protocole d'évaluation de la photostabilité des filtres

On évalue la photosatbilité du filtre UV par dosage HPLC du filtre résiduel après exposition au Suntest d'un film mince de formule à une dose d'UV équivalente à 1 H UVA radiométrique
Support d'étalement : PMMA dépoli
Quantité après application : 2 mg/cm²
Nombre d'échantillons : 4 plaques / formule exposés aux UV 4 plaques / formule témoins stockés à l'étuve à 37°C
Simulateur solaire :
   SUNTEST Heraeus - Atlas
   Flux UVA = 8,23.10⁻³ W/cm²
   Flux UVB = 1,42.10⁻³ W/cm²
   Temps d'exposition = 44 minutes
Les éclairements UVA et UVB du suntest sont mesurés radiométriquement. Extraction du film de crème : Ethanol + ultra sons
Dosage du filtre résiduel : HPLC
   Calcul du % de photostabilité relativement aux échantillons non exposés

### Résultats :

| **Exemples** | **SPF in vitro** | **Solubilité** | **% résiduel 2-hydrobenzophénone lipophile (Uvinul A+)** | **% résiduel de filtre UVB** |
|---|---|---|---|---|
| **Exemple A (invention)** | ++ | OUI | > 90% | > 90% |
| **Exemple B** | -- | OUI | > 90% | > 90% |
| **Exemple C** | -- | OUI | > 90% | > 90% |
| **Exemple D** | ++ | OUI | > 90% | Perte |
| **Exemple E** | ++ | NON | > 90% | > 90% |

Ces résultats montrent que seule l'association d'un filtre UVA de type 2-hydroxybenzophénone (Uvinul A Plus) et d'un filtre UVB choisi les s-triazines siliciées substituée par deux groupements aminobenzoates de formule (III) permet d'obtenir un système filtrant d'efficacité importante, aisé à mettre en oeuvre dans des compositions cosmétiques en raison de sa bonne solubilité et photostable. Pour les associations basées sur d'autres filtres UVB tels que la s-triazine siliciée substituée par deux groupements aminobenzoates d'alkyle en C₁₂, le α-cyano-β,β' diphénylacrylate de 2-éthylhexyle, le 4-méthoxycinnamate d'éthyl-2-hexyle, ou la 2,4,6-tris[p-(2'-éthylhexyl-1'-oxy-carbonyl)anilino]-1,3,5-triazine, les performances en terme d'efficacité filtrante, de solubilité ou de photostabilité sont inférieures à celles de l'association de l'invention.

### Exemples de formulation 1 à 6

| **Nom chimique** | **EX 1** | **EX 2** | **EX 3 (*)** | **EX 4 (*)** | **EX 5 (*)** | **EX 6 (*)** |
|---|---|---|---|---|---|---|
| **Phase a** | | | | | | |
| s-triazine siliciée substituée par deux groupements aminobenzoates Composé (5) | 2 | 2 | - | - | - | - |
| 2-(4-diéthylamino-2-hydroxybenzoyl)-benzoate de n-hexyle | 1,5 | 2,5 | 2,5 | 2,5 | 2,5 | 2,5 |
| Butyl Methoxy Dibenzoylmethane | 2 | 2 | 2 | 2 | 2 | 2 |
| s-triazine siliciée substituée par deux groupements aminobenzoates Composé (A) | | - | 2 | - | - | - |
| -cyano-β,β' diphénylacrylate de 2-éthylhexyle | - | - | - | 2 | | - |
| 4-méthoxycinnamate d'éthyl-2-hexyle | - | - | - | - | 2 | - |
| 2,4,6-tris[p-(2'-éthylhexyl-1'-oxy-carbonyl)anilino]-1,3,5-triazine | - | - | - | - | - | 2 |
| C₁₂-C₁₅ alkyl benzoate | 5 | 5 | 5 | 5 | 5 | 5 |
| Phenethyl benzoate (and) benzoic acid | 10 | 10 | 10 | 10 | 10 | 10 |
| Alcool cétylique | 0,5 | 0,5 | 0,5 | 0,5 | 0,5 | 0,5 |
| Acide stéarique | 1,5 | 1,5 | 1,5 | 1,5 | 1,5 | 1,5 |
| Mélange mono- stéarate de glycéryle / stéarate de PEG (100 OE) | 1 | 1 | 1 | 1 | 1 | 1 |
| Mélange de cétylstéaryl glucoside et d'alcools cétylique, stéarylique | 2 | 2 | 2 | 2 | 2 | 2 |
| Dimethicone | 0,50 | 0,50 | 0,50 | 0,50 | 0,50 | 0,50 |
| Triethanolamine | 0,45 | 0,45 | 0,45 | 0,45 | 0,45 | 0,45 |
| Conservateur | 1 | 1 | 1 | 1 | 1 | 1 |
| **Phase b** | | | | | | |
| Glycérol | 5 | 5 | 5 | 5 | 5 | 5 |
| Complexant | 0,1 | 0,1 | 0,1 | 0,1 | 0,1 | 0,1 |
| Phosphate de mono cétyle | 1 | 1 | 1 | 1 | 1 | 1 |
| Eau | qsp 100g | qsp 100g | qsp 100g | qsp 100g | qsp 100g | qsp 100g |
| **Phase c** | | | | | | |
| Gomme de Xanthane | 0,2 | 0,2 | 0,2 | 0,2 | 0,2 | 0,2 |
| Copolymère acide acrylique/méthacrylate de stéaryle | 0,2 | 0,2 | 0,2 | 0,2 | 0,2 | 0,2 |
| Iso-Hexadecane | 1 | 1 | 1 | 1 | 1 | 1 |
| Cyclopentasiloxane | | | 1 | 1 | 1 | 1 |
| **Phase d** | | | | | | |
| Triethanolamine | qs pH | qs pH | qs pH | qs pH | qs pH | qs pH |

### Mode opératoire

On chauffe la phase grasse a à 70°C. On chauffe la phase aqueuse b dans le bécher final. On prépare la phase c : dispersion des poudres dans l'huile. Sous agitation Rotor Stator, on émulsionne la phase grasse dans la phase aqueuse. On introduit la phase c sous agitation plus rapide puis laisser sous agitation lente jusqu'au retour à température ambiante. On neutralise la phase d et on conditionne.

## Revendications

1. Composition comprenant dans un support cosmétiquement acceptable au moins un système filtrant UV, **caractérisée par le fait qu'**elle comprend :
(i) au moins un filtre de type hydroxybenzophénone lipophile répondant à la formule (I) suivante : 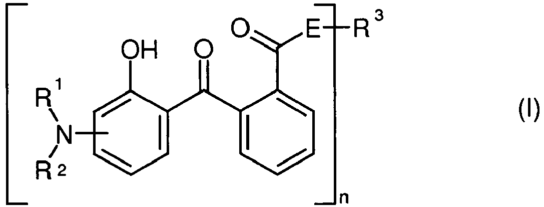 dans laquelle :
R¹ et R², identiques ou différents, désignent un radical alkyle en C₁-C₂₀, un alcényle en C₂-C₂₀, un cycloalkyle en C₃-C₁₀, un cycloalcényle en C₃-C₁₀ ou forment avec l'atome d'azote avec lequel ils sont liés un cycle à 5 ou 6 chaînons ;
n est un nombre allant de 1 à 4 avec
quand n = 1, R³ désigne un radical alkyle ou alcényle en C₁-C₂₀, un hydroxyalkyle en C₁-C₅, un cyclohexyle en C₆-C₁₂, un phényle pouvant être substitué par O, N ou S, aminocarbonyle ou alkylcarbonyle en C₁-C₅ ;
quand n = 2, R³ désigne un diradical alkyle, un diradical cycloalkyle, un diradical alcényle ou un diradical aryle ou R³ avec E forment un diradical de formule (II) : 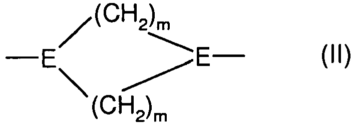
avec m un nombre allant de 1 à 3 ;
quand n = 3, R³ est un triradical alkyle ;
quand n = 4 , R³ est un tetraradical alkyle ;
E est -O-, ou -N(R⁴)- ;
R4 est hydrogène, un radical alkyle en C₁-C₅ ou hydroxyalkyle en C₁-C₅ ;
le ou les filtres lipophiles 2-hydroxybenzophénone étant présents à des teneurs variant de 0,1 à 10 % en poids par rapport au poids total de la composition ,
(ii) au moins une s-triazine siliciée de formule générale (III) suivante ou l'une de ses formes tautomères : 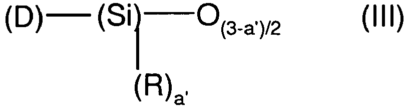 dans laquelle
- R, identiques ou différents représentent un radical alkyle en C₁-C₂, un radical phényle, un radical alkoxy en C₁-C₂, un radical hydroxy ou le groupe triméthylsilyloxy ;
- a'=1 à 3 ;
- le groupe (D) désigne un composé s-triazine de formule (IV) suivante : 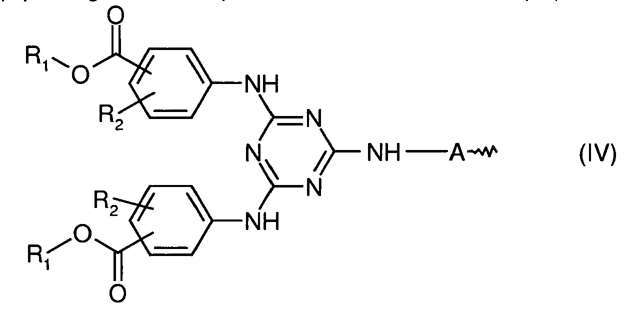 où
- R₁, identiques ou différents, représentent un radical alkyle en C₁-C₁₀, linéaire ou ramifié et éventuellement insaturé et pouvant contenir un groupe cycloalkyle en C₅-C₆,
- le groupement (C=O)OR₁ pouvant être en position ortho, méta ou para du groupement amino,
- R₂, identiques ou différents, représentent hydrogène, un radical hydroxy, un radical alkyle en C₁-C₄, linéaire ou ramifié, un radical méthoxy,
- A est un radical divalent choisi parmi méthylène ou un groupe répondant à l'une des formules (V), (VI), (VII) ou (VIII) suivantes : 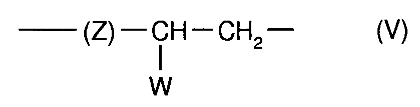 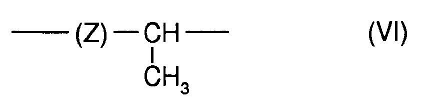
-(Z)-CH=CH- (VII) 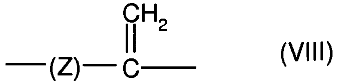
dans lesquelles :
- Z est un diradical alkylène en C₁-C₃,
- W représente un atome d'hydrogène, un radical hydroxyle ou un radical alkyle en C₁-C₃ ; en plus des unités de formule -A-(Si)(R)_{a'}(O)_{(3-a')/2}, l'organosiloxane peut comporter des unités de formule : (R)_{b}-(Si)(O)_{(4-b)/2} dans lesquelles :
R a la même signification que dans la formule (1), b = 1, 2 ou 3, ou
au moins une s-triazine siliciée répondant à l'une des formules (IIIa) ou (IIIb) suivantes : 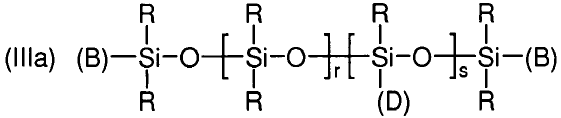 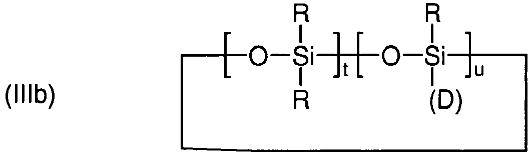 dans lesquelles :
- (D) répond à la formule (IV) telle que définie ci-dessus,
- R a la même définition que dans la formule (III) telle que définie ci-dessus,
- (B), identiques ou différents sont choisis parmi les radicaux R et le radical (D),
- r est un nombre entier compris entre 0 et 20 inclusivement,
- s est un nombre entier allant de 0 à 5 et si s = 0, au moins l'un des deux symboles (B) désigne (D),
- u est un nombre entier allant de 1 à 5,
- t est un nombre entier allant de 0 à 5, étant entendu que t + u est égal ou supérieur à 3 ainsi que leurs formes tautomères.

2. Composition selon la revendication 1, où l'hydroxybenzophénone de formule (I) est le 2-(4-diéthylamino-2-hydroxybenzoyl)-benzoate de n-hexyle de formule (a) :

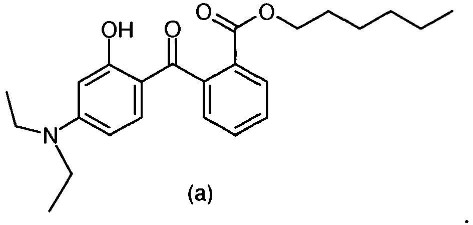

3. Composition selon la revendication, où l'hydroxybenzophénone de formule (I) est le (2-{4-[2-(4-diethylamino-2-hydroxy-benzoyl)-benzoyl]-piperazine-1-carbonyl}-phenyl)-(4-diethylamino-2-hydroxy-phenyl)-methanone de formule (b) :

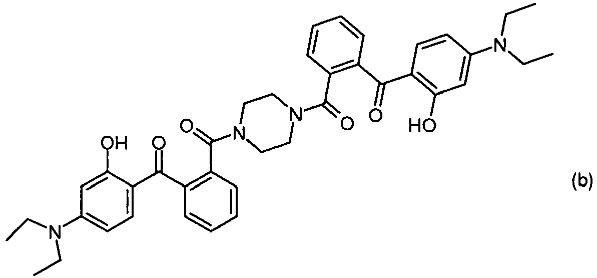

4. Composition selon l'une quelconque des revendications 1 à 3, où la s-triazine siliciée est choisie parmi celles pour lesquelles au moins l'une, et encore plus préférentiellement l'ensemble, des caractéristiques suivantes sont remplies :
R est méthyle,
a' = 1 ou 2,
R₁ est un radical en C₄-C₅,
Z = -CH₂-,
W=H.

5. Composition selon l'une quelconque des revendications 1 à 4, où la s-triazine siliciée répond à l'une des formules (IIIa) ou (IIIb) suivantes : 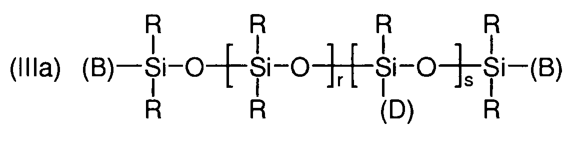 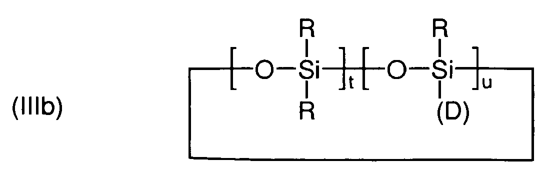 dans lesquelles :
- (D) répond à la formule (IV) telle que définie dans la revendication 1,
- R a la même définition que dans la formule (III) telle que définie dans la revendication 1,
- (B), identiques ou différents sont choisis parmi les radicaux R et le radical (D),
- r est un nombre entier compris entre 0 et 20 inclusivement,
- s est un nombre entier allant de 0 à 5 et si s = 0, au moins l'un des deux symboles (B) désigne (D),
- u est un nombre entier allant de 1 à 5,
- t est un nombre entier allant de 0 à 5, étant entendu que t + u est égal ou supérieur à 3 ainsi que leurs formes tautomères.

6. Composition selon la revendication 5, où la s-triazine siliciée est choisie parmi

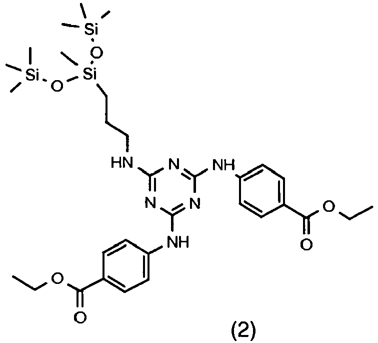

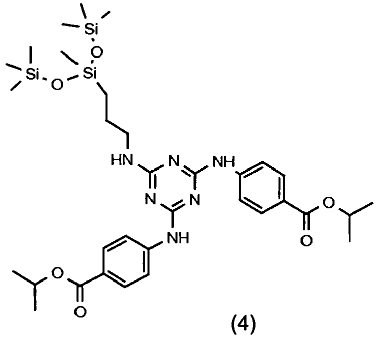

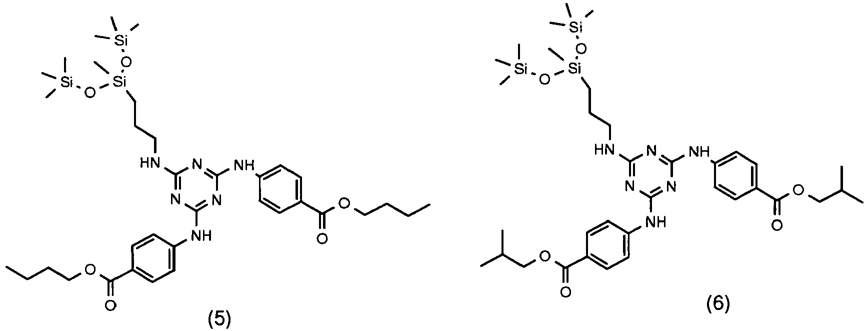

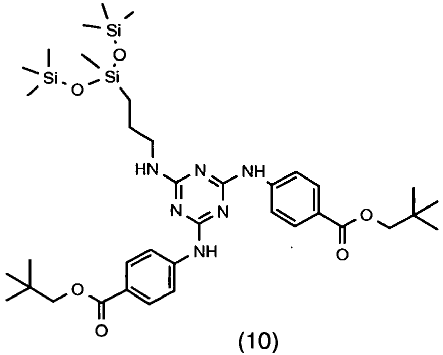

7. Composition selon la revendication 6, où la s-triazine siliciée est la 2,4-bis(4'-aminobenzoate de n-butyle)-6-[(3-{1,3,3,3-tetramethyl-1-[(trimethylsilyl)oxy]disiloxanyl}propyl)amino]-s-triazine de formule (5)

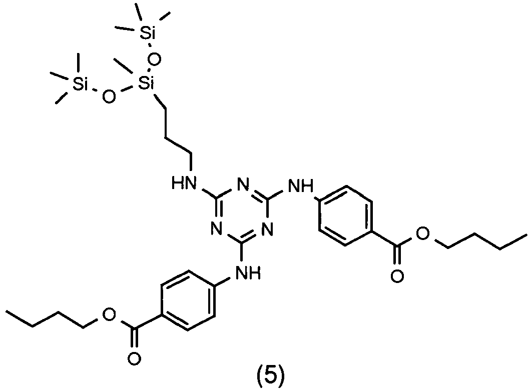

8. Composition selon l'une quelconques des revendications 1 à 7, comprenant en plus au moins un dérivé de dibenzoylméthane.

9. Composition selon l'une quelconque des revendications 1 à 8, **caractérisée par le fait qu'**elle contient en plus d'autres filtres organiques ou inorganiques actifs dans l'UV-A et/ou l'UV-B hydrosolubles ou liposolubles ou bien insolubles dans les solvants cosmétiques couramment utilisés.

10. Composition selon la revendication 9, où les filtres organiques complémentaires sont choisis parmi les anthranilates ; les dérivés cinnamiques ; les dérivés salicyliques, les dérivés du camphre ; les dérivés de la benzophénone hydrophiles ; les dérivés de β,β-diphénylacrylate ; les dérivés de triazine autres que ceux de formule (III) ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes ; les dérivés de mérocyanines et leurs mélanges.

11. Composition selon la revendication 9, **caractérisée par le fait que** le ou les filtres UV organiques sont choisis parmi les composés suivants :
Ethylhexyl Methoxycinnamate
Homosalate
Ethylhexyl Salicylate,
Octocrylene,
Phenylbenzimidazole Sulfonic Acid,
4-Methylbenzylidene camphor,
Ethylhexyl triazone,
Bis-Ethylhexyloxyphenol Methoxyphenyl Triazine
Diethylhexyl Butamido Triazone,
2,4,6-tris(biphenyl-4-yl-1,3,5-triazine
2,4,6-tris(4'-amino benzalmalonate de dinéopentyle)-s-triazine 2,4,6-tris-(4'-amino benzalmalonate de diisobutyle)-s- triazine,
2,4-bis(4'-amino benzalmalonate de dinéopentyle)-6-(4'-aminobenzoate de n-butyle)-s-triazine Methylène bis-Benzotriazolyl Tetramethylbutylphénol,
Polysilicone-15 Di-néopentyl 4'-méthoxybenzalmalonate 1,1-dicarboxy(2,2'-diméthyl-propyl)-4,4-diphénylbutadiène 2,4-bis-[5-1(diméthylpropyl)benzoxazol-2-yl-(4-phenyl)-imino]-6-(2-ethylhexyl)-imino-1,3,5-triazine et leurs mélanges.

12. Composition selon la revendication 9, **caractérisée par le fait que** les filtres inorganiques complémentaires sont des pigments d'oxydes métalliques, enrobés ou non.

13. Procédé de photostabilisation vis-à-vis du rayonnement d'au moins un filtre du type dérivé du dibenzoylméthane par une composition comprenant au moins un filtre UV de type 2-hydroxybenzophénone lipophile tel que défini dans les revendications précédentes en une teneur variant de 0,1 à 10 % en poids par rapport au poids de la composition, et au moins une s-triazine siliciée substituée par au moins deux groupements aminobenzoates de formule (III) telle que définie dans les revendications précédentes.

## Patentansprüche

1. Zusammensetzung, umfassend, in einem kosmetisch verträglichen Träger, mindestens ein UV-Filtersystem, **dadurch gekennzeichnet, dass** es Folgendes umfasst:
(i) mindestens einen lipophilen Hydroxybenzophenon-Filter, der der folgenden Formel (I) gehorcht: 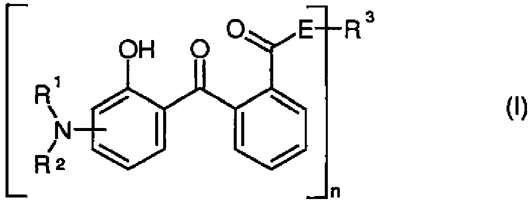
wobei R¹ und R², gleich oder verschieden, einen C₁-C₂₀-Alkylrest, einen C₂-C₂₀-Alkenylrest, einen C₃-C₁₀-Cycloalkylrest, einen C₃-C₁₀-Cycloalkenylrest bezeichnen oder mit dem Stickstoffatom, an das sie gebunden sind, einen 5- oder 6-gliedrigen Ring bilden;
n eine Zahl von 1 bis 4 ist, wobei
wenn n = 1, R³ einen C₁-C₂₀-Alkyl- oder C₁-C₂₀-Alkenylrest, einen C₁-C₅-Hydroxyalkylrest, einen C₆-C₁₂-Cyclohexylrest bezeichnet, wobei ein Phenyl mit O, N oder S, Aminocarbonyl oder C₁-C₅-Alkylcarbonyl substituiert sein kann;
wenn n = 2, R³ einen diradikalischen Alkylrest, einen diradikalischen Cycloalkylrest, einen diradikalischen Alkenylrest oder einen diradikalischen Arylrest bezeichnet oder R³ mit E einen diradikalischen Rest der folgenden Formel (II) bildet: 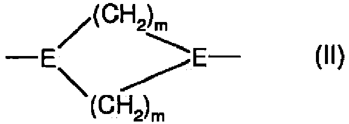
wobei m eine Zahl von 1 bis 3 ist;
wenn n = 3, R³ ein triradikalischer Alkylrest ist;
wenn n = 4, R³ ein tetraradikalischer Alkylrest ist;
E -O- oder -N(R⁴)- ist;
R⁴ Wasserstoff, ein C₁-C₅-Alkylrest oder ein C₁-C₅-Hydroxyalkylrest ist, wobei der oder die lipophilen 2-Hydroxybenzophenon-Filter in einem Gehalt von 0,1 bis 10 Gew.-%, bezogen auf das Gesamtgewicht der Zusammensetzung, vorhanden sind,
(ii) mindestens ein siliziertes s-Triazin der folgenden allgemeinen Formel (III) oder eine seiner tautomeren Formen: 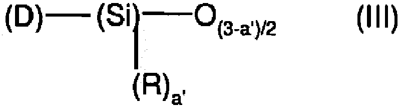 wobei
- R, gleich oder verschieden, einen C₁-C₂-Alkylrest, einen Phenylrest, einen C₁-C₂-Alkoxyrest, einen Hydroxyrest oder die Trimethylsilyloxy-Gruppe darstellt;
- a' = 1 bis 3;
- die Gruppe (D) eine s-Triazin-Verbindung der folgenden Formel (IV) bezeichnet: 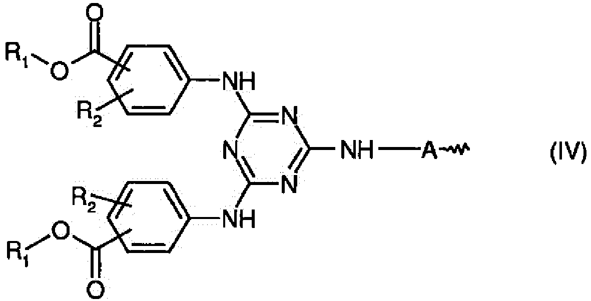 wobei
- R₁, gleich oder verschieden, einen linearen oder verzweigten und gegebenenfalls ungesättigten- C₁-C₁₀-Alkylrest darstellt und eine C₅-C₆-Cycloalkylgruppe enthalten kann,
- die Gruppe (C=O)OR₁ in Ortho-, meta- oder Para-Position der Aminogruppe vorhanden sein kann,
- R₂, gleich oder verschieden, Wasserstoff, einen Hydroxyrest, einen C₁-C₄-Alkylrest, einen linearen oder verzweigten C₁-C₄-Alkylrest oder Methoxyrest darstellt;
- A einen zweiwertigen Rest darstellt, der ausgewählt ist aus Methylen oder einer Gruppe, die einer der Formeln (V), (VI), (VII) oder (VIII) gehorcht: 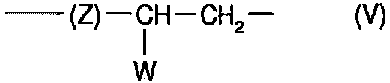 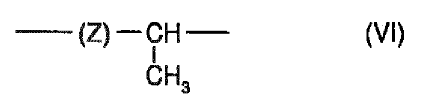
-(Z)-CH=CH- (VII) 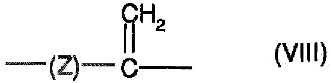
wobei:
- Z ein zweiwertiger C₁-C₃-Alkylenrest ist,
- W ein Wasserstoffatom, einen Hydroxylrest oder einen C₁-C₃-Alkylrest darstellt; weiterhin Einheiten der Formel -A-(Si)(R)_{a'}(O)(_{3-a')/2}, das Organosiloxan kann Einheiten der Formel (R)_{b}-(Si)(O)_{(4-b)/2} umfassen, wobei R die gleiche Bedeutung besitzt wie in der Formel (I), b = 1,2 oder 3, oder
mindestens ein siliziertes s-Triazin einer der folgenden Formeln (IIIa) oder (IIIb) gehorcht: 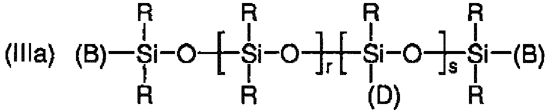 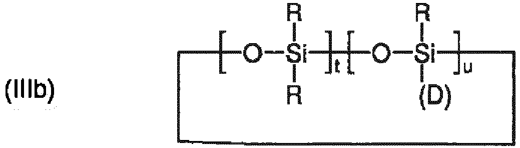 wobei:
- (D) der Formel (IV) gehorcht, wie zuvor definiert,
- R die gleiche Bedeutung wie in der Formel (III) besitzt, wie zuvor definiert,
- (B), gleich oder verschieden, ausgewählt ist aus den Resten R und dem Rest (D),
- r eine ganze Zahl von 0 bis 20, Grenzen eingeschlossen, ist,
- s eine ganze Zahl von 0 bis 5 ist, und wenn s = 0, mindestens eines der beiden Symbole (B) (D) bezeichnet,
- u eine ganze Zahl von 1 bis 5 ist,
- t eine ganze Zahl von 0 bis 5 ist, mit der Maßgabe, dass t + u gleich oder größer 3 ist, sowie ihre tautomeren Formen.

2. Zusammensetzung nach Anspruch 1, wobei das Hydroxybenzophenon der Formel (I) 2-(4-Diethylamino-2-hydroxybenzoyl)-n-hexylbenzoat der Formel (a) ist:

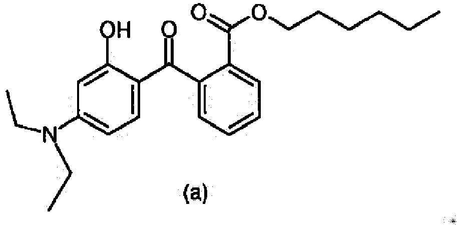

3. Zusammensetzung nach Anspruch 1, wobei das Hydroxybenzophenon der Formel (I) (2-{4-[2-(4-Diethylamino-2-hydroxy-benzoyl)-benzoyl]-piperazin-1-carbonyl]-phenyl)-(4-diethylamino-2-hydroxy-phenyl)-methanon der Formel (b) ist:

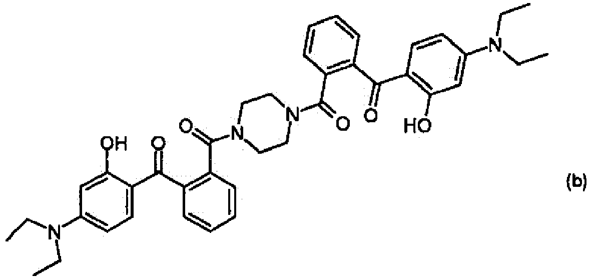

4. Zusammensetzung nach einem der Ansprüche 1 bis 3, wobei das silizierte s-Triazin aus denjenigen ausgewählt ist, wofür mindestens ein und noch stärker bevorzugt die Gesamtheit der folgenden Merkmale erfüllt sind:
R ist Methyl,
a' = 1 oder 2,
R₁ ist ein C₄-C₅-Rest
Z = -CH₂-,
W=H.

5. Zusammensetzung nach einem der Ansprüche 1 bis 4, wobei das silizierte s-Triazin einer der folgenden Formeln (IIIa) oder (IIIb) gehorcht: 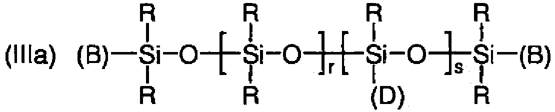 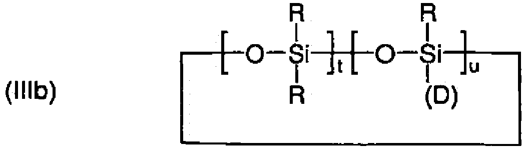 wobei:
- (D) der Formel (IV) gehorcht, wie in Anspruch 1 definiert,
- R die gleiche Definition wie in der Formel (III) aufweist, wie in Anspruch 1 definiert,
- (B), gleich oder verschieden, aus den Resten R und dem Rest (D) ausgewählt ist,
- r eine ganze Zahl von 0 bis 20, Grenzen eingeschlossen, ist
- s eine ganze Zahl von 0 bis 5 ist, und wenn s = 0, mindestens eine der beiden Symbole (B) (D) bezeichnet,
- u eine ganze Zahl von 1 bis 5 ist,
- t eine ganze Zahl von 0 bis 5 ist, mit der Maßgabe, dass t + u gleich oder größer 3 ist, sowie ihre tautomeren Formen.

6. Zusammensetzung nach Anspruch 5, wobei das silizierte s-Triazin ausgewählt ist aus

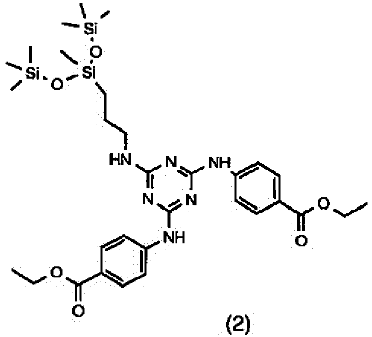

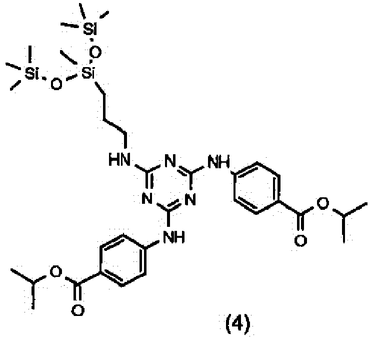

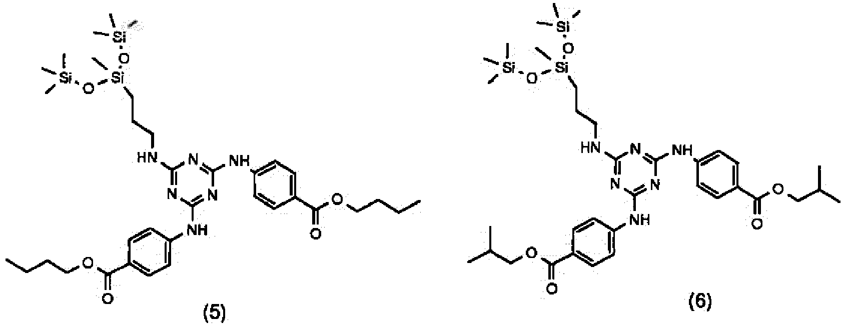

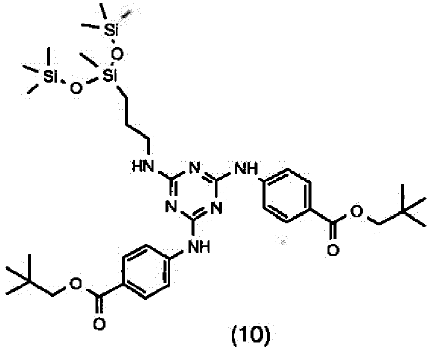

7. Zusammensetzung nach Anspruch 6, wobei das silizierte S-Triazin 2,4-bis(n-butyl-4'-aminobenzoat)-6-[(3-{1,3,3,3-tetramethyl-1-[(trimethylsilyl)oxy]disiloxanyl}propyl)amino]-s-triazin- der Formel (5) ist

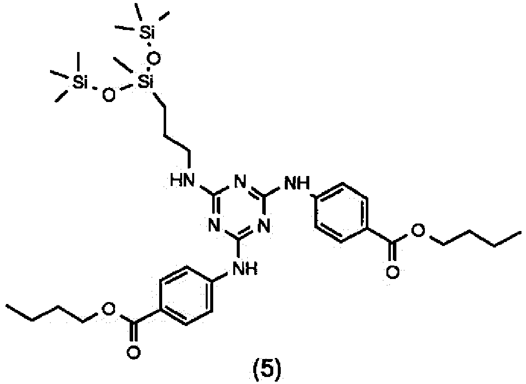

8. Zusammensetzung nach einem der Ansprüche 1 bis 7, weiterhin umfassend mindestens ein Dibenzoylmethanderivat.

9. Zusammensetzung nach einem der Ansprüche 1 bis 8, **dadurch gekennzeichnet, dass** sie weiterhin andere organische oder anorganische UV-A- und/oder UV-B-wirksame wasser- oder fettlösliche Filter, oder auch in derzeit verwendeten kosmetischen Lösungsmitteln unlösliche Filter enthält.

10. Zusammensetzung nach Anspruch 9, wobei die organischen zusätzlichen Filter ausgewählt sind aus Anthranilaten; Zimtsäurederivaten, Bernsteinsäurederivaten, Campherderivaten, hydrophilen Benzophenonderivaten; β-β-Diphenylacrylatderivaten; Triazinderivaten, die anders sind als diejenigen der Formel (III); Benzotriazolderivaten; Benzalmalonatderivaten; Benzimidazolderivaten; Imidazolinen; Bis-benzazolyldeuvaten; Aminobenzoesäurederivaten (PABA); Methylen-bis(hydroxyphenylbenzotriazol)-Derivaten; Benzoxazolderivaten; Polymer- und Siliconfiltern, dimeren α-Alkylstyrolderivaten 4,4-Diarylbutadienen; Derivaten von Merocyaninen und ihren Gemischen.

11. Zusammensetzung nach Anspruch 9, **dadurch gekennzeichnet, dass** der oder die organischen UV-Filter ausgewählt sind aus den folgenden Verbindungen:
Ethylhexalmethoxycinnamat
Homosalat
Ethylhexylsalicylat,
Octocrylen,
Phenylbenzimidazolsulfonsäure,
4-Methylbenzylidenkampher,
Ethylhexyltriazon,
Bis-ethylhexyloxyphenolmethoxyphenyltriazin
Diethylhexyl-butamido-triazon,
2,4,6-Tris(biphenyl-4-yl-1,3,5-triazin
2,4,6-Tris(4'-dineopentyl-aminobenzalmalonat)-s-triazin
2,4,6-Tris(4'-diisobutyl-aminobenzalmalonat)-s-triazin
2,4-Bis(44'-dineopentylaminobenzalmalonat)-6-(n-butyl-4'-aminobenzoat)-s-triazin
Methylen-bis-benzotriazolyltetramethylbutylphenol
Polysilicon-15
Di-neopentyl-4'-methoxybenzalmalonat
1,1-Dicarboxy-(2,2'-dimethyl-propyl)-4,4-diphenylbutadien
2,4-Bis[5-1(dimethylpropyl)benzoxazol-2-yl-(4-phenyl)-imino]-6-(2-ethylhexyl)-imino-1,3,5-triazin
und ihren Gemischen.

12. Zusammensetzung nach Anspruch 9, **dadurch gekennzeichnet, dass** die zusätzlichen anorganischen Filter beschichtete oder nicht beschichtete Metalloxidpigmente sind.

13. Verfahren zur Photostabilisierung gegenüber Strahlung mit mindestens einem Dibenzoylmethanderivat-Filter durch eine Zusammensetzung, umfassend mindestens einen UV-Filter vom Typ lipophiles 2-Hydroxybenzophenon, wie in den vorangehenden Ansprüchen definiert, wobei ein Gehalt von 0,1 bis 10 Gew.-%, bezogen auf das Gewicht der Zusammensetzung, variiert, und mindestens ein siliziertes s-Triazin, das mit mindestens zwei Amino-benzoat-Gruppierungen der Formel (III), wie in den vorangehenden Ansprüchen definiert, substituiert ist.

## Claims

1. A composition comprising in a cosmetically acceptable support at least one UV-filtering system, **characterized by** the fact that it comprises:
(i) at least one filter of the lipophilic hydroxybenzophenone type corresponding to the following formula (I): 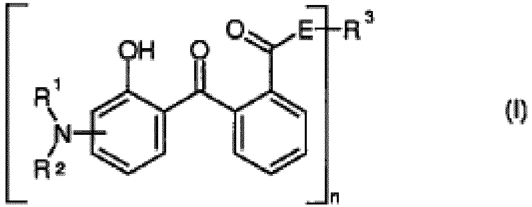 wherein:
R¹ and R², either identical or different, designate a C₁-C₂₀ alkyl radical, a C₂-C₂₀ alkenyl, a C₃-C₁₀ cycloalkyl, a C₃-C₁₀ cycloalkenyl or form with the nitrogen atom to which they are bound, a ring with 5 or 6 members;
n is a number ranging from 1 to 4 with
when n=1, R³ designates a C₁-C₂₀ alkyl or alkenyl radical, a C₁-C₅ hydroxyalkyl, a C₆-C₁₂ cyclohexyl, a phenyl which may be substituted with 0, N or S, aminocarbonyl or C₁-C₅ alkylcarbonyl;
when n=2, R³ designates an alkyl diradical, a cycloalkyl diradical, an alkenyl diradical or an aryl diradical or R³ with E form a diradical of formula (II): 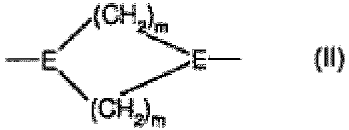
with m being a number from 1 to 3;
when n=3, R³ is an alkyl triradical;
when n=4, R³ is a alkyl tetraradical;
E is -O-, or -N(R⁴)-;
R4 is a hydrogen, a C₁-C₅ alkyl or C₁-C₅ hydroxyalkyl;
the lipophilic hydroxybenzophenonefilter(s) being present in contents varying from 0.1 to 10% by weight based on the total weight of the composition,
(ii) at least one siliceous s-triazine of the following general formula (III) or one of its tautomeric forms: 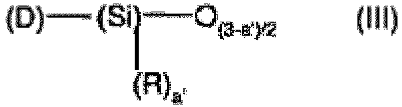
wherein
- R, either identical or different, represent a C₁-C₂ alkyl radical, a phenyl radical, a C₁-C₂ alkoxy radical, a hydroxyl radical or the trimethylsilyloxy group;
- a'=1 to 3;
- the group (D) designates an s-triazine compound of the following formula (IV): 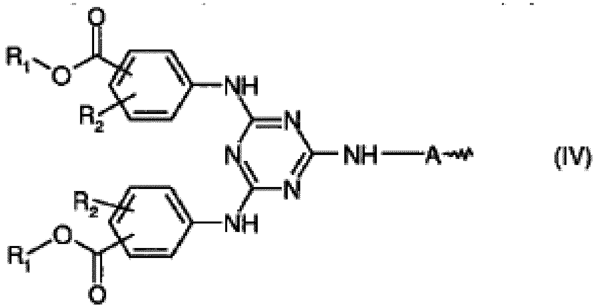 wherein
- R₁, either identical or different, represent a linear or branched, optionally unsaturated C₁-C₁₀ alkyl radical and which may contain a C₅-C₆ cycloalkyl group
- the (C=O)OR₁ group which may be in the ortho, meta or para of the amino group,
- R₂, either identical or different, represent a hydrogen, a hydroxy radical, a linear or branched C₁-C₄ alkyl radical, a methoxy radical,
- A is a divalent radical selected from methylene or a group fitting one of the following formulae (V), (VI), (VII) or (VIII): 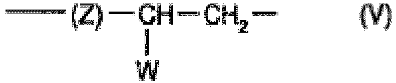 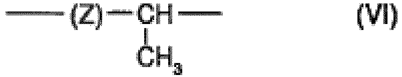 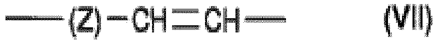 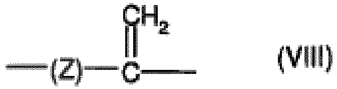
wherein:
- Z is a C₁-C₃ alkylene diradical,
- W represents a hydrogen atom, a hydroxyl radical or a C₁-C₃ alkyl radical;
in addition to units of formula -A-(Si)(R)_{a'}(O)_{(3-a')/2}, the organosilane may include units of formula: (R)_{b}-(Si)(O)_{(4-b)/2} wherein:
R has the same meaning as in formula (I), b=1, 2 or 3, or
at least one siliceous s-triazine fitting one of the following formulae (IIIa) or (IIIb): 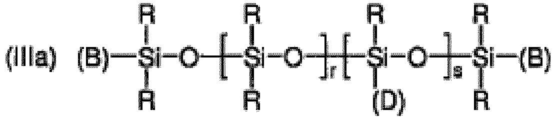 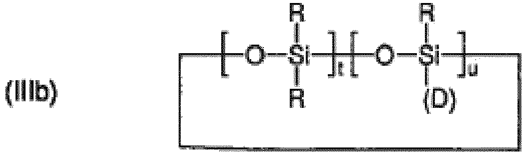
wherein:
- (D) fits formula (IV) as defined above,
- R has the same definition as in formula (III), as defined above,
- (B), either identical or different, are selected from the radicals R and the radical (D)
- r is an integer comprised between 0 and 20 inclusively,
- s is an integer ranging from 0 to 5 and if s=0, at least one of the two symbols (B) refers to (D),
- u ist ain integrer ranging from 1 to 5,
- t is an integer ranging from 0 to 5, it being understood that t+u is equal to or greater than 3 as well as their tautomeric forms.

2. The composition according to claim 1, wherein the hydroxybenzophenone of formula (I) is the n-hexyl 2-(4-diethylamino-2-hydroxybenzoyl)-benzoate of formula (a) :

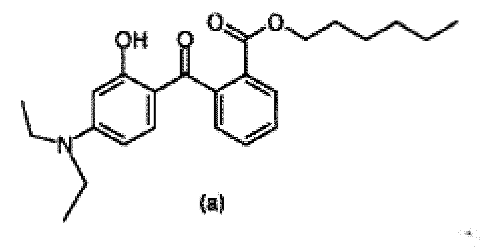

3. The composition according to claim 1, wherein the hydroxybenzophenone of formula (I) is the (2-{4-[2-(4-diethylamino-2-hydroxy-benzoyl)-benzoyl]-piperazine-1-carbonyl}-phenyl)-(4-diethylamino-2-hydroxy-phenyl)-methanone of formula (b):

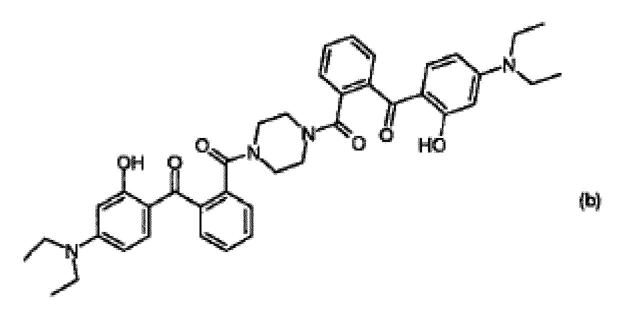

4. The composition according to any of claims 1 to 3, wherein the siliceous s-triazine is selected from those for which at least one, and even more preferentially the whole, of the following characteristics are fulfilled:
R is a methyl,
a' = 1 or 2,
R₁ is a C₄-C₅ radical,
Z = -CH₂-,
W = H.

5. The composition according to any of claims 1 to 4, wherein the siliceous s-triazine fits one of the following formulae (IIIa) or (IIIb): 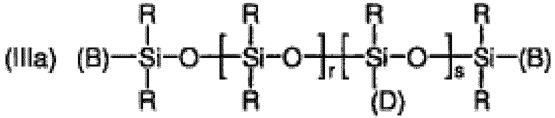 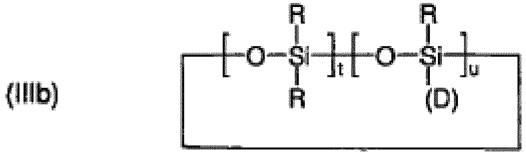 wherein:
- (D) fits formula (IV) as defined in claim 1,
- R has the same definition as in formula (III) as defined in claim 1,
- (B), either identical or different, are selected from the radicals R and the radical D,
- r is an integer comprised between 0 and 20 inclusively,
- s is an integer ranging from 0 to 5 and if s=0, at least one of the two symbols (B) refers to (D),
- u is an integer ranging from 1 to 5,
- t is an integer ranging from 0 to 5, it being understood that t+u is equal to or greater than 3 as well as their tautomeric forms.

6. The composition according to claim 5, wherein the siliceous s-triazine is selected from among

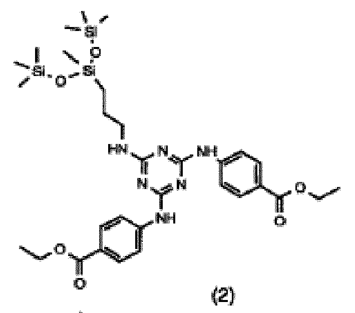

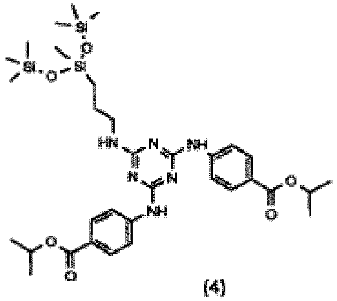

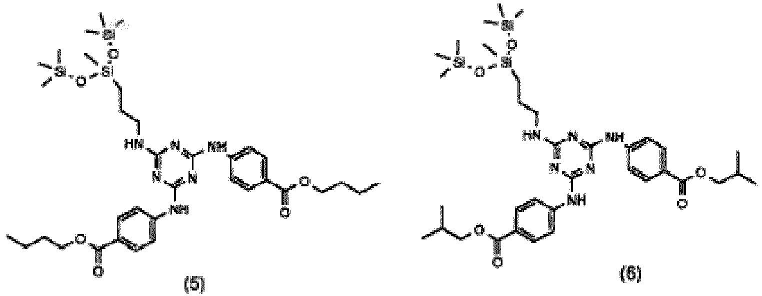

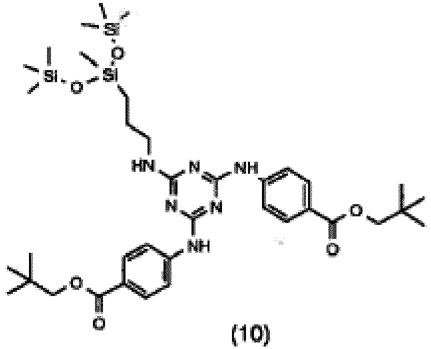

7. The composition according to claim 6, wherein the siliceous s-triazine is the 2,4-bis(n-butyl 4'aminobenzoate)-6[(3-{1,3,3,3-tetramethyl-1-[(trimethylsilyl)oxy]-disiloxanyl}propyl)amino]-s-triazine of formula (5)

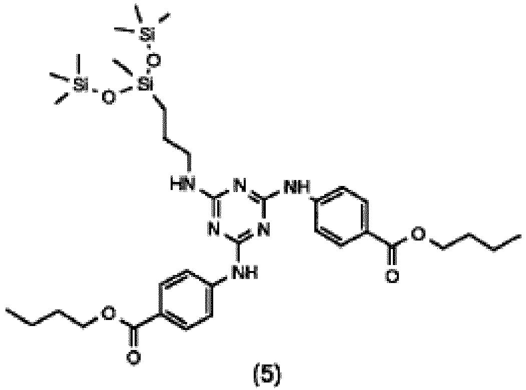

8. The composition according to any of claims 1 to 7, further comprising at least one dibenzoylmethane derivative.

9. The composition according to any of claims 1 to 8, further containing other UV-A and/or UV-B active organic or inorganic filters, either water-soluble or fat-soluble, or else insoluble in commonly used cosmetic solvents.

10. The composition according to claim 9, wherein the additional organic filters are selected from among anthranilates; cinnamic derivatives; salicylic derivatives; camphor derivatives; hydrophilic benzophenone derivatives; β,β-diphenyl acrylate derivatives; triazine derivatives other than those of formula (III); benzotriazole derivatives; benzalmalonate derivatives; benzimidazole derivatives; imidazolines; bis-benzoazolyl derivatives; p-aminobenzoic acid (PABA) derivatives; bis-(hydroxyphenyl benzotriazole) methylene derivatives; benzoxazole derivatives; filter polymers and filter silicones; dimers derived from α-alkylstyrene; 4,4-diarylbutadienes; merocyanine derivatives and mixtures thereof.

11. The composition according to claim 9, **characterized by** the fact that the organic UV filters are selected from the following compounds:
Ethylhexyl methoxycinnamate
Homosalate
Ethylhexyl salicylate,
Octocrylene,
Phenylbenzimidazole sulfonic acid,
4-methylbenzylidene camphor,
Ethylhexyl triazone,
Bis-ethylhexyloxyphenol methoxyphenyl triazine,
Diethylhexyl butamido triazone,
2,4,6-tris(biphenyl-4-yl)-1,3,5-triazine 2,4,6-tris(dineopentyl 4'-aminobenzalmalonate)-s-triazine
2,4,6-tris(diisobutyl 4'-aminobenzalmalonate)-s-triazine
2,4-bis(dineopentyl 4'-aminobenzalmalonate)-6-(n-butyl 4'-aminobenzalmalonate)-s-triazine Methylene bis-benzotriazolyl tetramethylbutylphenol,
Polysilicone-15 Di-neopentyl 4'-methoxybenzalmalonate 1,1-dicarboxy(2,2-dimethylpropyl)-4,4-diphenylbutadiene 2,4-bis-[5-1(dimethylpropyl)benzoxazol-2-yl-(4-phenyl)-imino]-6-(2-ethylhexyl)-imino-1,3,5-triazine and mixtures thereof.

12. The composition according to claim 9, **characterized by** the fact that the additional inorganic filters are pigments of metal oxides either coated or not.

13. A method for photostabilization towards radiation of at least one filter derived from dibenzoylmethane with a composition comprising at least one lipophilic UV filter of the 2-hydroxybenzophenone type as defined in the preceding claims in a content varying from 0.1 to 10% by weight based on the weight of the composition, and at least one siliceous s-triazine substituted with at least two aminobenzoate groups of formula (III) as defined in the preceding claims.
